Europäisches Patentamt
European Patent Office
Office européen des brevets

(11) Veröffentlichungsnummer: **0 279 781 A2**

(12) # EUROPÄISCHE PATENTANMELDUNG

(21) Anmeldenummer: **88810083.1**

(22) Anmeldetag: **11.02.88**

(51) Int. Cl.4: **C 07 D 205/08**
C 07 D 307/32,
C 07 D 307/68,
C 07 C 101/30,
C 07 C 103/48, C 07 F 7/18
// C07C143/68

(30) Priorität: **17.02.87 CH 577/87**

(43) Veröffentlichungstag der Anmeldung:
**24.08.88 Patentblatt 88/34**

(84) Benannte Vertragsstaaten:
**BE CH DE ES FR GB IT LI NL SE**

(71) Anmelder: **CIBA-GEIGY AG**
**Klybeckstrasse 141**
**CH-4002 Basel (CH)**

(72) Erfinder: **Sedelmeier, Gottfried, Dr.**
**Erlenweg 11**
**D-7801 Schallstadt (DE)**

**Bersier, Jacques, Dr.**
**Gstaltenrainweg 61**
**CH-4125 Riehen (CH)**

(54) **Verfahren zur Herstellung von 4-Acetoxy-3-hydroxyethyl-azetidinon.**

(57) Die Erfindung betrifft neue Verfahren zur Herstellung von 4-Acetoxy-3-hydroxyethyl-azetidinon der Formel

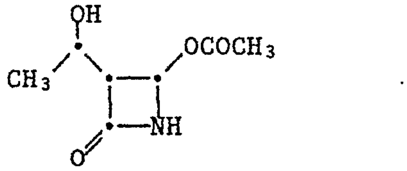

(I),

insbesondere von 4(R)-Acetoxy-3(R)-(1'(R)-hydroxyethyl)-2-azetidinon, aus enantiomerenreinen Verbindungen der Formel

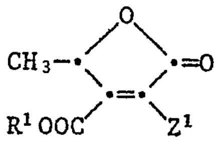

(II),

worin $R^1$ Niederalkyl und $Z^1$ Amino, Arylmethylamino, Acylamino oder Azido bedeuten, durch Reduktion der C=C-Doppelbindung und gegebenenfalls der Arylmethylamino- oder Azidogruppe mit Wasserstoff, Lactonringöffnung, Ringschluss zum β-Lactam, gegebenenfalls nach Hydrolyse der Acylaminogruppe, und oxidativer Decarboxylierung der ursprünglichen Lacton-Carboxygruppe in Gegenwart eines Acetatabgebenden Mittels. Verbindungen der Formel I können als Ausgangsstoffe zur Herstellung von β-Lactam-Antibiotika verwendet werden. Neue Zwischenprodukte und Ausgangsmaterialien sind ebenfalls Gegenstand der Erfindung.

EP 0 279 781 A2

**Beschreibung**

Verfahren zur Herstellung von 4-Acetoxy-3-hydroxyethyl-azetidinon

Die Erfindung betrifft neue Verfahren zur Herstellung von 4-Acetoxy-3-hydroxyethyl-azetidinon, die als Ausgangsstoffe zur Herstellung von β-Lactam-Antibiotika verwendet werden können. Neue Zwischenprodukte und Ausgangsmaterialien sind ebenfalls Gegenstand der Erfindung.

Das nach dem erfindungsgemässen Verfahren herstellbare 4-Acetoxy-3-hydroxyethyl-azetidinon der Formel I, insbesondere das 4(R)-Acetoxy-3(R)-(1'(R)-hydroxyethyl)-2-azetidinon der Formel Ia,

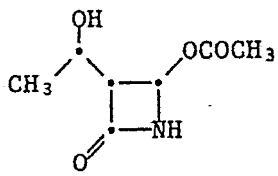 (I) 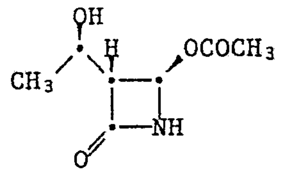 (Ia)

ist als Ausgangsmaterial für die Herstellung einer Vielzahl von hochwirksamen β-Lactam-Antibiotika, beispielsweise von Penemen, Carbapenemen oder entsprechenden Oxapenem-, Penicillin- oder Cephalosporin-Derivaten geeignet. Bei diesen Umsetzungen wird die Acetoxygruppe in 4-Stellung des Azetidinons durch geeignete Schwefel- oder Kohlenstoff-Nukleophile ausgetauscht. Solche Reaktionen sind beispielsweise im Uebersichtsartikel von W. Dürckheimer et al., Angew. Chem. 97, 183 (1985), erläutert.

Zu Penemen gelangt man beispielsweise, indem man die Verbindung der Formel I, gegebenenfalls nach Einführung einer Schutzgruppe an der Hydroxygruppe und/oder dem Lactam-Stickstoffatom, mit Mercaptanen, Thiosäuren, Dithiosäuren, Trithiocarbonaten oder verwandten Verbindungen umsetzt, das Stickstoffatom mit einem geeigneten Essigsäurederivat alkyliert und schliesslich den schwefelhaltigen Fünfring schliesst. Zum Aufbau von Carbapenemen wird beispielsweise die Verbindung der Formel I oder ein geschütztes Derivat dieser Verbindung mit einem geeignet substituierten Enolsilylether, Zinnenolat, Borenolat, Tetraallylzinn oder einer verwandten Verbindung umgesetzt und die erhaltenen Produkte entsprechend weiterverarbeitet.

Wesentliche Kriterien für die antibiotische Wirkung von Penemen und Carbapenemen sind nicht nur Art und Stellung, sondern auch die räumliche Anordnung der Substituenten. Das Diastereomere der Formel Ia weist eine günstige räumliche Anordnung der Hydroxy-, Hydroxyethyl- und Acetoxygruppe auf, um als Ausgangsmaterial für antibiotisch wirksame β-Lactam-Antibiotika zu dienen. Aber auch andere Diastereomere sind für die Weiterverarbeitung geeignet: Die an C(4) epimere cis-Verbindung ergibt bei der Substitutionsreaktion mit Schwefel- und Kohlenstoff-Nukleophilen unter geeigneten Reaktionsbedingungen ebenfalls die in der Verbindung der Formel Ia schon vorhandene trans-Anordnung der Substituenten am Azetidinon-Ring. Die an C(1') epimere (S)-Hydroxyethyl-Verbindung kann verwendet werden, weil auf einer späteren Stufe eine Umwandlung in eine (R)-Hydroxyethyl-Verbindung durch Substitution mit Inversion erreicht werden kann.

Die Herstellung der Verbindung der Formel I ist bekannt. In EP 78026 und Tetrahedron Letters 23, 2293-2296 (1982) ist beispielsweise ein Verfahren beschrieben, bei dem die Verbindung der Formel Ia aus L-Asparaginsäure über Acylierung oder Hydroxyalkylierung des Dianions einer N-geschützten Azetidinon-4-carbonsäure hergestellt wird. EP 106652 beschreibt ein Verfahren, bei welchem eine Schiff'sche Base von Glyoxylsäure mit Diketen umgesetzt, die Acetylgruppe in der Seitenkette zu Hydroxyethyl reduziert und eine Enantiomerentrennung über diastereomere Ester vorgenommen wird. In EP 171064 wird ein Prozess beschrieben, bei dem ein 4-Ethynylazet idinon durch Cycloaddition aufgebaut wird und anschliessend der Ethynylrest in Acetyl und schliesslich durch Baeyer-Villiger-Reaktion in Acetoxy umgewandelt wird. EP 181831 beschreibt ein Verfahren, bei welchem ein α,β-Epoxybutyryl-acetonylamid mit Base cyclisiert und das gebildete 3-Hydroxyethyl-4-acetylazetidinon zur 4-Acetoxy-Verbindung aufoxidiert wird.

Beschreibung des Verfahrens

Die Erfindung betrifft Verfahren zur Herstellung von diastereomerenreinen Verbindungen der Formel

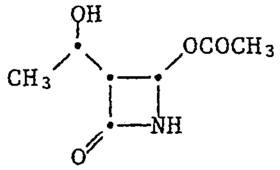 (I),

insbesondere des (3R, 4R, 1'R)-Diastereomeren der Formel Ia, dadurch gekennzeichnet, dass man eine enantiomerenreine Verbindung der Formel

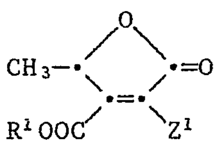

(II),

worin $R^1$ Niederalkyl und $Z^1$ Amino, Arylmethylamino, Acylamino oder Azido bedeuten, oder ein Salz davon mit Wasserstoff reduziert und gewünschtenfalls eine Acylaminogruppe $Z^1$ und/oder die Niederalkoxycarbonylgruppe $COOR^1$ hydrolysiert, in einer erhältlichen Verbindung der Formel

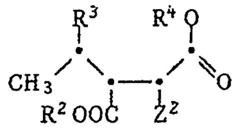

(III),

worin $R^2$ Wasserstoff oder Niederalkyl, $R^3$ und $R^4$ zusammen eine Bindung und $Z^2$ Amino oder Acylamino bedeuten, oder in einem Salz davon, gewünschtenfalls nach Umwandlung der Reste $R^2$ und $Z^2$ und/oder nach Umwandlung von Diastereomeren ineinander, mit einer Base den Lactonring öffnet und, falls $Z^2$ Acylamino bedeutet, die Acylaminogruppe $Z^2$ hydrolysiert,
in einer erhältlichen Verbindung der Formel III, worin $R^2$ Wasserstoff oder Niederalkyl, $R^3$ Hydroxy, $R^4$ Wasserstoff und $Z^2$ Amino bedeuten, oder in einem Salz davon $R^3$ und $COOR^4$ und, wenn $R^2$ Wasserstoff bedeutet, auch $COOR^2$ mit einer Schutzgruppe schützt, mit einer starken Base den β-Lactamring schliesst und die Schutzgruppen abspaltet, und
eine erhältliche Verbindung der Formel

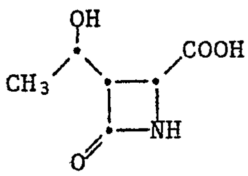

(IV)

oder ein Salz davon oxidativ in Gegenwart eines Acetat-abgebenden Mittels decarboxyliert.

Gegenüber den vorbekannten Verfahren weist das neue erfindungsgemässe Verfahren wesentliche Vorteile auf, können doch damit die Verbindung der Formel I und deren Diastereomere mit hoher Gesamtausbeute, hoher Stereoselektivität, wenig Reaktionsschritten und einfachen und preisgünstigen Reagentien hergestellt werden. Ueberraschenderweise ist es möglich, ausgehend von enantiomerenreinen Verbindungen der Formel II mit nur einem chiralen Kohlenstoffatom durch Wahl der Reduktionsbedingungen als auch durch allfällige nachträgliche Isomerisierung zu reinen Diastereomeren der Formel III mit drei chiralen Kohlenstoffatomen zu gelangen. Es ist auch überraschend, dass die Reaktionssequenz zur Herstellung der Verbindung I, die empfindliche funktionelle Gruppe enthält, nur gerade für die Bildung des β-Lactamringes eine Schutzgruppe benötigt, wobei diese erst noch leicht in situ eingeführt und wieder abgespalten werden kann. Ueberraschend ist weiter, dass reine Diastereomere der Formel I in wenigen Reaktionsschritten aus einem Ausgangsmaterial der Formel II erhältlich sind, wenn man bedenkt, dass dieses Ausgangsmaterial einfach aus billigen enantiomerenreinen Naturprodukten aufgebaut oder durch Enantiomerentrennung von racemischem synthetischem Material gewonnen werden kann.

Die in der Definition von Substituenten verwendeten allgemeinen Ausdrücke und Bezeichnungen haben vorzugsweise die folgenden Bedeutungen:

Nieder, z.B. in Niederalkyl, Niederalkoxy oder Niederalkanoyl, bedeutet, dass die so bezeichneten Reste und Gruppen 1 bis 7 C-Atome und bevorzugt 1 bis 4 C-Atome enthalten.

Niederalkyl $R^1$ oder $R^2$ hat vorzugsweise 1 bis 7 C-Atome und ist z.B. Methyl, Ethyl, n-Propyl, Isopropyl, n-Butyl oder tert-Butyl.

Arylmethylamino $Z^1$ ist beispielsweise Benzylamino, Dibenzylamino, 2-, 3- oder 4-Methylbenzylamino, α- oder β-Naphthylmethylamino oder Diphenylmethylamino.

Acylamino $Z^1$ oder $Z^2$ ist beispielsweise unsubstituiertes oder substituiertes Niederalkanoylamino, z.B. Formylamino, Acetylamino, Propionylamino, tert-Butylcarbonylamino, Chloracetylamino, Trichloracetylamino oder Trifluoracetylamino, Niederalkoxycarbonylamino, z.B. tert-Butoxycarbonylamino, Arylniederalkoxycarbonylamino, z.B. Benzyloxycarbonylamino, 4-Nitrobenzyloxycarbonylamino oder Diphenylmethoxycarbonylamino, oder Arylcarbonylamino, z.B. Benzoylamino.

Eine Carboxyschutzgruppe $R^2$ oder $R^4$ oder die Schutzgruppe von geschütztem Hydroxy $R^3$ ist beispielsweise eine der Schutzgruppen, wie sie in Standardwerken, z.B. in J.F.W. McOmie, "Protective Groups in Organic Chemistry", Plenum Press London und New York 1973, in Th. W. Greene, "Protective Groups in

Organic Synthesis", Wiley, New York 1981, in "The Peptides"; Band 3 (Herausg. E. Gross und J. Meienhofer), Academic Press, London und New York 1981, sowie in "Methoden der organischen Chemie", Houben-Weyl, 4. Auflage, Bd. 15/I, Georg Thieme Verlag, Stuttgart 1974, beschrieben sind.

Bevorzugte Schutzgruppen sind beispielsweise solche Gruppen, die gleichzeitig an der Hydroxygruppe und der (den) Carboxygruppe(n) eingeführt werden können, die genannten funktionellen Gruppen vor einer Reaktion mit nicht oder nur wenig nukleophilen basischen Reagentien schützen und durch Hydrolyse unter milden Bedingungen wieder abgespalten werden. Als Carboxyschutzgruppen $R^2$ oder $R^4$ kommen in erster Linie Triniederalkylsilylreste, z.B. Trimethylsilyl, Tributylsilyl, Triisopropylsilyl, tert-Butyldimethylsilyl oder 1,1,2-Trimethylpropyl-dimethyl-silyl, vorzugsweise Trimethylsilyl, aber auch arylsubstituierte Silylreste, z.B. Dimethylphenylsilyl oder Triphenylsilyl, in Betracht. Weiter bevorzugt sind in 1-Stellung verzweigte und/oder substituierte Niederalkylreste, beispielsweise tert-Niederalkyl, z.B. tert-Butyl, Di- oder Triarylmethyl, z.B. Diphenylmethyl, Di(p-methoxyphenyl)methyl oder Trityl, Niederalkoxyniederalkyl, z.B. Methoxymethyl, 1-Methoxyethyl oder 1-Ethoxyethyl, oder Niederalkylthioniederalkyl, z.B. Methylthiomethyl oder 1-Ethylthioethyl, ferner die entsprechenden cyclischen Gruppen 2-Oxa- oder 2-Thiacycloalkyl mit 5-7 Ringatomen, z.B. 2-Tetrahydrofuryl oder 2-Tetrahydropyranyl.

Geschütztes Hydroxy $R^3$ ist bevorzugt ein der obigen Definition von $R^2$ und $R^4$ entsprechender -oxy-Rest, beispielsweise Triniederalkylsilyloxy, z.B. Trimethylsilyloxy, arylsubstituiertes Silyloxy, z.B. Triphenylsilyloxy, in 1-Stellung verzweigtes und/oder substituiertes Niederalkoxy, z.B. tert-Butoxy oder Methoxymethoxy, oder 2-Oxa- oder 2-Thiacycloalkoxy, z.B. 2-Tetrahydropyranyloxy.

Salze sind beispielsweise Säureadditionssalze der Aminogruppe in Verbindung der Formel II oder III, z.B. mit anorganischen Säuren, z.B. Salzsäure, Schwefelsäure, Salpetersäure oder Phosphorsäure, oder mit organischen Carbon- oder Sulfonsäuren, z.B. mit Essigsäure, Chloressigsäure, Trichloressigsäure, Trifluoressigsäure, Propion säure, Glykolsäure, Fumarsäure, Benzoesäure, Methansulfonsäure, Trifluormethansulfonsäure, Ethansulfonsäure, Campher-10-sulfonsäure, Benzolsulfonsäure, Toluolsulfonsäure, 4-Nitrobenzolsulfonsäure, 2,4-Dinitrobenzolsulfonsäure oder Naphthalin-2-sulfonsäure.

Carbonsäuren der Formeln III und IV können Alkalimetall-, z.B. Natrium-oder Kaliumsalze bilden, ferner Erdalkalimetallsalze, z.B. Magnesium- oder Calciumsalze, Schwermetallsalze, z.B. Kupfer-, Blei-oder Zinksalze, Ammoniumsalze, Salze mit organischen Aminen, z.B. mit gegebenenfalls substituierten Mono-, Di- oder Trialkylaminen, z.B. mit Cyclohexylamin, Diethylamin, Cyclohexylethylamin, Dibutylamin, Trimethylamin, Triethylamin oder Tri-(2-hydroxyethyl)-amin, oder mit tetrasubstituierten organischen Ammoniumionen, z.B. mit Tetramethylammonium, Tetraethylammonium oder Tetrabutylammonium.

Verbindungen der Formel III, in denen $R^2$ Wasserstoff und/oder $R^4$ Wasserstoff und $Z^2$ Amino bedeuten, können auch innere Salze bilden.

Die Verbindung der Formel I besitzt drei voneinander unabhängige chirale Kohlenstoffatome, nämlich Kohlenstoffatome 3 und 4 des β-Lactamringes und das die Hydroxygruppe tragende Kohlenstoffatom 1′ der Hydroxyethyl-Seitenkette. Somit umfasst die Darstellung der Formel I acht diastereomere Verbindungen.

Das erfindungsgemässe Verfahren betrifft die Herstellung aller dieser diastereomeren Verbindungen in reiner Form. Besonders bevorzugt ist die Herstellung des (3R,4S,1′R)- und des (3R,4R,1′R)-Diastereomeren der Formel I, insbesondere des (3R,4R,1′R)-Diastereomeren der Formel Ia, aus dem (R)-Enantiomeren der Verbindung der Formel II.

Im Zusammenhang mit der vorliegenden Beschreibung heisst enantiomerenrein oder diastereomerenrein, dass das entsprechende Enantiomere beziehungsweise Diastereomere zu mindestens 80 % im Gemisch mit dem anderen Enantiomeren beziehungsweise den anderen Diastereomeren vorliegt. Dieser hohe Anteil eines Stereoisomers erlaubt, durch einfache physikalische Trennmethoden, z.B. durch Umkristallisation einer erfindungsgemässen Verbindung, eines geeigneten Salzes oder eines geeigneten Derivates, den Anteil des Stereoisomeren auf beispielsweise über 95 % oder so zu erhöhen, dass die andern Stereoisomeren mit den üblichen analytischen Methoden nicht mehr nachgewiesen werden können.

Verbindungen der Formel II, worin $Z^1$ Amino, Arylmethylamino oder Acylamino bedeuten, stehen im Gleichgewicht mit der tautomeren Form der Formel

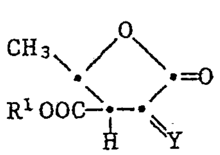 (IIa),

worin Y Imino, Arylmethylimino oder Acylimino bedeutet. Das Tautomerengleichgewicht ist abhängig von der Art der Substituenten $R^1$ und $Z^1$/Y und den physikalischen Bedingungen, z.B. Aggregatzustand, Lösungsmittel, pH, Druck und Temperatur, und kann im kristallinen Zustand in der Formel II oder IIa eingefroren sein.

Im folgenden schliesst die Bezeichnung "Verbindung der Formel II" auch die tautomere Form der Formel IIa mit ein.

Verfahrensschritte

Die Reduktion einer Verbindung der Formel II, worin $Z^1$ Amino, Arylmethylamino, Acylamino oder Azido bedeutet, oder eines Salzes davon kann mit Wasserstoff in Gegenwart eines der üblichen Hydrierkatalysatoren durchgeführt werden.

Solche für die Reduktion geeigneten Hydrierkatalysatoren sind vorzugsweise Edelmetallkatalysatoren, z.B. Platin, Palladium, Ruthenium oder Rhodium, in feinverteilter metallischer Form, beispielsweise aufgebracht auf einen inerten Träger mit grosser Oberfläche, z.B. auf Aktivkohle, Alox, Bariumsulfat oder andere Erdalkalimetallsalze. Bevorzugt sind die besonders für die Reduk tion von C=C-Doppelbindungen geeigneten Edelmetall-Hydrierkatalysatoren, beispielsweise Platin, wie es durch Reduktion von Platinoxid mit Wasserstoff erhalten wird, Palladium auf Kohle, Rhodium auf Alox oder eine Platin-Rhodium-Legierung nach Nishimura. Weiter geeignet als Hydrierkatalysator ist Nickel, z.B. Raney-Nickel, wie es beim Zersetzen einer Nickel-Aluminium-Legierung mit Alkali gebildet wird, ferner lösliche Edelmetall-Komplexkatalysatoren, z.B. Rhodium-Phosphinkomplexe oder Iridium-Phosphinkomplexe.

Die Hydrierungen werden in einem inerten Lösungsmittel durchgeführt, beispielsweise in einem Alkohol, z.B. Methanol, Ethanol oder Isopropanol, in Alkohol-Wasser-Mischungen, z.B. wässrigem Ethanol, wobei der Alkohol auch Mineralsäure, z.B. Salzsäure, enthalten kann, in Carbonsäuren, z.B. Essigsäure oder Propionsäure, auch wässriger Essigsäure, in polaren Estern, z.B. Essigsäureethylester, in Kohlenwasserstoffen, z.B. Toluol oder Cyclohexan, oder in Halogenkohlenwasserstoffen, z.B. Methylenchlorid. Vorzugsweise werden die Hydrierungen in einem Temperaturbereich von 0° bis 100°C, z.B. bei Raumtemperatur oder leicht erhöhter Temperatur bis ca. 80°C und bei Normaldruck, leicht erhöhtem Wasserstoffdruck bis ungefähr 5 bar oder hohem Wasserstoffdruck bis ungefähr 100 bar in Abhängigkeit des Substituenten $Z^1$ und des gewählten Katalysators durchgeführt. Beispielsweise wird mit Palladium auf Kohle vorzugsweise bei 1-5 bar, mit Platin, Rhodium oder Platin-Rhodium-Legierung bei hohem Druck, vorzugsweise 50-100 bar, hydriert.

Wird eine Verbindung, worin $Z^1$ Arylmethylamino oder Azido bedeutet, hydriert, so kann die Reaktion einstufig oder zweistufig über die entsprechende Verbindung der Formel II, worin $Z^1$ Amino bedeutet, geführt werden, weil eine Arylmethylaminogruppe und eine Azidgruppe leichter als die C=C-Kohlenstoffdoppelbindung hydriert werden. Vorzugsweise wird die Arylmethylaminogruppe, z.B. Benzylaminogruppe, oder die Azidgruppe in Gegenwart von Palladium auf Kohle bei Raumtemperatur oder leicht erhöhter Temperatur bei einem Wasser stoffdruck von 1-5 bar, vorzugsweise Normaldruck, in einem der genannten Lösungsmittel, beispielsweise in salzsaurem Ethanol oder in Essigsäureethylester, selektiv hydriert.

Eine Verbindung der Formel II, worin $Z^1$ Amino bedeutet, oder ein Salz davon kann direkt zu einer Verbindung der Formel III hydriert werden. Andrerseits kann die Aminogruppe auch vor der Hydrierung in eine Acylaminogruppe $Z^1$ übergeführt werden. Die Acylierung erfolgt unter den üblichen Reaktionsbedingungen, beispielsweise unter Verwendung des der Acylgruppe entsprechenden Säureanhydrids oder Säurehalogenids, z.B. Säurechlorids, gegebenenfalls unter Zugabe einer Säure, z.B. p-Toluolsulfonsäure, oder einer Base, z.B. eines tertiären Amins, z.B. Triethylamin, Dimethylbenzylamin oder N-Methylmorpholin, eines Amidins, z.B. 1,5-Diazabicyclo[4.3.0]non-5-en oder 1,8-Diazabicyclo[5.4.0]undec-7-en, einer Pyridinbase, z.B. Pyridin oder Lutidin, oder eines Alkali- oder Erdalkalicarbonats, z.B. Natrium-, Kalium- oder Calciumcarbonat, in einem Ueberschuss des Acylierungsmittels oder in einem inerten Lösungsmittel, beispielsweise in einem Ether, z.B. Diethylether, Tetrahydrofuran oder Dioxan, einem Halogenkohlenwasserstoff, z.B. Methylenchlorid, einem Ester, z.B. Essigsäureethylester, einem Amid, z.B. Dimethylformamid oder N-Methylpyrrolidon, oder in einem Nitril, z.B. Acetonitril, bei Temperaturen zwischen -20°C und dem Siedepunkt des Lösungsmittels, vorzugsweise zwischen 0° und 50°C.

Nach der Reduktion einer Verbindung der Formel II, worin $Z^1$ Acylamino bedeutet, kann gewünschtenfalls die Acylaminogruppe in eine entsprechende Aminogruppe umgewandelt werden. Die Hydrolyse wird unter den üblichen Bedingungen erreicht, beispielsweise mit Säure oder Base.

Geeignete Säuren sind beispielsweise Mineralsäuren, z.B. Salzsäure, Schwefelsäure oder Phosphorsäure, oder starke organische Säuren, z.B. Alkan- oder Arylsulfonsäuren, z.B. Methansulfonsäure, Trifluormethansulfonsäure, Benzolsulfonsäure, p-Toluolsulfonsäure oder p-Nitrobenzolsulfonsäure, vorzugsweise in wässriger Lösung, bei spielsweise in Wasser oder in Mischungen von Wasser und organischen Lösungsmitteln, z.B. Methanol, Ethanol, Dioxan oder Acetonitril, bei Temperaturen zwischen 20° und 150°C, vorzugsweise 50° bis 100°C. Bedeuten $R^3$ Hydroxy und $R^4$ Wasserstoff, so werden diese unter den genannten sauren Bedingungen gleichzeitig abgespalten und in eine Bindung übergeführt.

Geeignete Basen sind Metallhydroxide, beispielsweise Alkalimetallhydroxide, z.B. Lithium-, Natrium- oder Kaliumhydroxid, Alkalimetallcarbonate, z.B. Natrium- oder Kaliumcarbonat, oder tetrasubstituierte Ammoniumhydroxide, z.B. Benzyltrimethylammoniumhydroxid, vorzugsweise in Wasser oder den obengenannten Mischungen von Wasser und organischen Lösungsmitteln bei Temperaturen zwischen 20° und 120°C, z.B. um 50°C. Der basischen Hydrolyse kann zur Beschleunigung Wasserstoffperoxid, z.B. als 30% wässrige Lösung, zugesetzt werden. Bei der basischen Hydrolyse wird gleichzeitig der Lactonring geöffnet und eine Verbindung der Formel III erhalten, worin $R^3$ Hydroxy und $R^4$ Wasserstoff bedeuten. Diese Reste $R^3$ und $R^4$ können durch Behandlung mit einer Säure, beispielsweise mit einer der obengenannten Säuren, wieder abgespalten und in eine Bindung übergeführt werden.

Die obengenannten sauren oder basischen Hydrolysebedingungen für eine Acylaminogruppe $Z^1$ bewirken üblicherweise gleichzeitig auch eine Hydrolyse der Niederalkylestergruppe $COOR^1$, sodass Verbindungen der Formel III gebildet werden, in denen $R^2$ Wasserstoff bedeutet. Soll nach der Hydrierung einer Verbindung der

Formel II selektiv die Niederalkylestergruppe $COOR^1$ hydrolysiert werden, so genügen auch schon mildere Reaktionsbedingungen. Die Esterspaltung gelingt beispielsweise mit den obengenannten Säuren und Basen in wässriger Lösung bei niedrigeren Temperaturen, z.B. zwischen 0°C und Raumtemperatur. Es können auch starke organische Carbonsäuren, z.B. Chlor-, Trichlor- oder Trifluoressigsäure, oder organische Amine, z.B. Trialkylamin, z.B. Triethylamin oder Tributylamin, oder Pyridin, in wässriger Lösung verwendet werden.

Soll im nächsten Schritt eine Verbindung der Formel III, in der $R^2$ Wasserstoff ist und $R^3$ und $R^4$ zusammen eine Bindung dargstellen, in eine entsprechende Verbindung, worin $R^2$ Niederalkyl bedeutet, umgewandelt werden, so verwendet man dazu die üblichen Veresterungsbedingungen. Geeignet sind beispielsweise ein Ueberschuss des entsprechenden Niederalkanols $R^2$ OH in Gegenwart einer Säure, beispielsweise einer möglichst wasserfreien Mineralsäure, z.B. Chlorwasserstoff, konzentrierte Schwefelsäure, Polyphosphorsäure oder Phosphorpentoxid, einer organischen Sulfonsäure, z.B. p-Toluolsulfonsäure, eines sauren Ionenaustauschers oder einer Lewis-Säure, z.B Bortrifluoridetherat, gewünschtenfalls in Gegenwart eines wasserentziehenden Mittels, beispielsweise Molekularsieb, oder unter Entfernung des Reaktionswassers durch azeotrope Destillation, beispielsweise mit einem Halogenkohlenwasserstoff, z.B. Methylenchlorid, Chloroform oder Tetrachlorkohlenstoff, oder einem aromatischen Kohlenwasserstoff, z.B. Benzol oder Toluol. Die saure Veresterung wird beispielsweise bei Temperaturen zwischen 0°C und dem Siedepunkt des Alkohols $R^2OH$ oder des Azeotrops mit dem genannten Schlepper-Lösungsmittel durchgeführt. Eine Veresterung kann auch mit einem Alkylierungsmittel erreicht werden, z.B. mit Diazomethan in etherischer Lösung.

Zur Umwandlung einer Verbindung der Formel III, in der $Z^2$ Amino ist und $R^3$ und $R^4$ zusammen eine Bindung darstellen, in eine entsprechende Verbindung, worin $Z^2$ Acylamino bedeutet, verwendet man die weiter oben für Verbindungen der Formel II genannten Acylierungsbedingungen. Gleichermassen können die weiter oben genannten Hydrolysebedingungen verwendet werden, wenn in einer Verbindung der Formel III $Z^2$ mit der Bedeutung Acylamino in $Z^2$ mit der Bedeutung Amino und/oder wenn $R^2$ mit der Bedeutung Niederalkyl in $R^2$ mit der Bedeutung Wasserstoff umgewandelt werden soll.

In einer Verbindung der Formel III, worin $R^2$ Niederalkyl und $R^3$ und $R^4$ zusammen eine Bindung darstellen, kann durch geeignete Basen eine Umwandlung von reinen Diastereomeren oder von Diastereomerengemischen in Diastereomere mit all-trans-Anordnung der Substituenten $CH_3$, $COOR^2$ und $Z^2$ am Lactonfünfring erreicht werden. Je nach der durch die Wahl des enantiomerenreinen Ausgangsmaterials der Formel II vorgegebenen Konfiguration des Kohlenstoffatoms C(4), das die Methylgruppe trägt, entsteht bei dieser Umwandlung das (2S,3S,4R)- oder das (2R,3R,4S)-Diastereomere. Geeignete Basen für eine solche Isomerisierung sind beispielsweise tertiäre Amine, z.B. Triethylamin oder Tributylamin, Pyridin oder Pyridinderivate, z.B. Lutidin, Amidinbasen, z.B. 1,5-Diazabicyclo[4.3.0]non-5-en oder 1,8-Diazabicyclo[5.4.0]undec-7-en, oder basische Erdalkalimetallsalze, z.B. Natrium- oder Kaliumcarbonat oder Kaliumfluorid. Die Isomerisierung wird vorzugsweise in polaren, wasserfreien Lösungsmitteln, beispielsweise Methanol, Ethanol, Tetrahydrofuran, Dioxan, Acetonitril, Dimethylformamid oder Dimethylsulfoxid bei Temperaturen zwischen 0° und 50°C, beispielsweise um Raumtemperatur, durchgeführt.

Im nächsten Schritt wird in einer Verbindung der Formel III, worin $R^3$ und $R^4$ zusammen eine Bindung darstellen, durch Behandeln mit einer wässrigen Base, beispielsweise wässrigem Alkalimetallhydroxid, z.B. Lithium-, Natrium- oder Kaliumhydroxid, oder Alkalimetallcarbonat, z.B. Natrium- oder Kaliumcarbonat, gewünschtenfalls in Gegenwart eines Lösungsvermittelnden organischen Lösungsmittels, z.B. Methanol, Ethanol oder Ethylenglykol, vorzugsweise bei Temperaturen zwischen 0°C und Raumtemperatur, der Lactonring geöffnet. Es ist auch möglich, die weiter oben unter Hydrolyse der Acylaminogruppe $Z^2$ angegebenen basischen Reaktionsbedingungen anzuwenden. Das bei der Basenbehandlung gebildete Salz, z.B. Alkalimetallsalz, der Verbindung der Formel III, worin $R^3$ Hydroxy und $R^4$ Wasserstoff bedeuten, wird mit einer äquimolaren Menge einer Protonen liefernden Säure, beispielsweise einer Mineralsäure, z.B. Salzsäure, angesäuert. Falls $Z^2$ Acylamino bedeutet, ist gleichzeitig oder im Anschluss an die Oeffnung des Lactonringes mit einer wässrigen Base auch die Acylaminogruppe $Z^2$ in der weiter oben angegebenen Art und Weise zu hydrolysieren.

Im nächsten Schritt werden in der Verbindung der Formel III die Hydroxygruppe $R^3$, die Carboxygruppe $COOR^4$ und, wenn $R^2$ Wasserstoff ist, auch die Carboxygruppe $COOR^2$ durch geeignete Schutzgruppen geschützt. Einführung und Abspaltung der Schutzgruppen sind in den oben zitierten Standardwerken über Schutzgruppen beschrieben.

Eine Silylschutzgruppe, z.B. Trimethylsilyl, wird durch das entsprechende Silylhalogenid, z.B. Iodid, Bromid oder vorzugsweise Chlorid, in Gegenwart einer Base, z.B. Pyridin, Dimethylaminopyridin, Imidazol, Triethylamin, Dicyclohexylamin oder Di(trimethylsilyl)amin, über das entsprechende Silylamid, z.B. Trimethylsilylacetamid oder 3-(Trimethylsilyl)-2-oxazolidinon, oder mit anderen reaktiven Silylderivaten eingeführt. Die entsprechenden Halogenide in Gegenwart der genannten Basen sind auch geeignet zur Einführung von tert-Niederalkyl-, Diarylmethyl-, Triarylmethyl-, Niederalkoxymethyl- oder Niederalkylthiomethyl-Schutzgruppen. tert-Niederalkylgruppen und in 1-Stellung durch Sauerstoff oder Schwefel substituierte Reste werden vorzugsweise über die entsprechenden Olefine, z.B. Isobutylen, Ethyl-vinyl-ether, 2,3-Dihydrofuran oder 3,4-Dihydro-2H-pyran, in Gegenwart wasserfreier Säuren, beispielsweise Mineralsäure, z.B. Chlorwasserstoff, Sulfonsäure, z.B. p-Toluolsulfonsäure, oder Lewis-Säure, z.B. Bortrifluoridetherat, eingeführt. Zur Einführung der Schutzgruppe verwendet man vorzugsweise wasserfreie, inerte Lösungsmittel, beispielsweise Kohlwasserstoffe, z.B. Toluol, Halogenkohlenwasserstoffe, z.B. Methylenchlorid, oder Ether, z.B. Diethylether oder Tetrahydrofuran, bei Temperaturen zwischen 0°C und dem Siedepunkt des Lösungsmittls, z.B. um

Raumtemperatur. Um eine Reaktion der Aminogruppe mit dem die Schutzgruppe einführenden Reagens zu vermeiden, ist die Reaktion unter kontrollierten Bedingungen, beispielsweise mit äquivalenten Mengen des Reagenses, durchzuführen und/oder anschliessend die Aminogruppe wieder freizusetzen, beispielsweise durch Reaktion mit Ammoniak oder einem primären Amin, z.B. Methylamin.

Die Verbindung der Formel III, in der $R^3$ geschütztes Hydroxy, $COOR^4$ geschütztes Carboxy und $COOR^2$ Niederalkoxycarbonyl oder geschütztes Carboxy bedeuten, wird mit einer starken Base zu einem β-Lactam cyclisiert. Geeignete starke Basen sind jene, die bevorzugt die Aminofunktion deprotonieren, ohne gleichzeitig die geschützte(n) Carboxygruppe(n) und gegebenenfalls Niederalkoxycarbonyl $COOR^2$ zu verändern. In Frage kommen beispielsweise nicht-nukleophile Alkalimetallamide, beispielsweise Lithiumdiisopropylamid, Lithiumcyclohexylethylamid, Lithium-2,2,6,6-tetramethylpiperidid oder Kalium-bis(trimethylsilyl)amid, oder in 1-Stellung verzweigte Niederalkyllithium- oder Niederalkylmagnesium-Verbindungen, beispielsweise tert-Butyllithium, Isopropylmagnesiumhalogenid oder vorzugsweise tert-Butylmagnesiumhalogenid, z.B. tert-Butylmagnesiumchlorid, in inerten Lösungsmitteln, beispielsweise Ether, z.B. Diethylether oder Tetrahydrofuran, Kohlenwasserstoffen, z.B. Toluol, Pentan, Hexan oder Cyclohexan, oder Gemischen der genannten Lösungsmittel, bei Temperaturen zwischen -80° und Raumtemperatur, beispielsweise um 0°C.

Das Reaktionsgemisch wird in schwach saurer wässriger Lösung bei einem pH zwischen 2 und 5 aufgearbeitet, beispielsweise in wässriger verdünnter, gewünschtenfalls gepufferter Mineralsäure, z.B. Phosphorsäure, Carbonsäure, z.B. Essigsäure, oder in schwach saurer Salzlösung, z.B. Kaliumhydrogensulfat oder Kalium- oder Natriumhydrogentartrat, wobei gleichzeitig die Hydroxy- und Carboxyschutzgruppen abgespalten werden.

Die auf diese Weise erhältliche Verbindung der Formel IV wird durch Bleitetraacetat oder vorzugsweise elektrochemisch in Gegenwart eines Acetat-abgebenden Mittels oxidativ decarboxyliert.

Zur chemischen oxidativen Decarboxylierung der Verbindung der Formel IV wird diese mit einer äquimolaren Menge oder einem Ueberschuss an Bleitetraacetat, vorzugsweise mit 1 bis 1,5 Aequivalenten, in einem inerten, polaren Lösungsmittel bei Temperaturen zwischen 20° und 80°C, beispielsweise um 50°C umgesetzt, beispiels weise in Essigsäure, Dimethylformamid, N-Methylpyrrolidon, Hexamethylphosphorsäuretriamid, Tetrahydrofuran, Dioxan oder dergleichen, vorzugsweise in Lösungsmittelgemischen, z.B. Essigsäure/Dimethylformamid, und beispielsweise unter Zusatz von Alkalimetallacetaten, z.B. Natrium- oder Kaliumacetat, und/oder Aminen, z.B. Pyridin oder Lutidin.

Zur elektrochemischen oxidativen Decarboxylierung verwendet man eine Monozelle oder eine geteilte Zelle mit einem mechanischen Diaphragma, z.B. aus porösem Ton oder Glas oder aus Polyvinylchlorid, oder mit einem Ionenaustauscher-Diaphragma, beispielsweise wie unter dem Handelsnamen Nafion® erhältlich, und Elektroden aus Edelmetall, beispielsweise Platin, Titanlegierungen, z.B. Titan-Iridium oder Titan-Tantal, ferner Nickel, Bleidioxid, Glaskohlenstoff und/oder Graphit. Die elektrochemische Decarboxylierung wird in Gegenwart eines Acetat-abgebenden Mittels, z.B. in Essigsäure oder Gemischen von Essigsäure und inerten organischen Lösungsmitteln, beispielsweise Acetonitril, Dioxan oder Dimethylformamid durchgeführt, wobei ein Amin, beispielsweise Triniederalkylamin, z.B. Triethylamin oder Tri-n-butylamin, oder Pyridin, und/oder Alkalimetallacetate, beispielsweise Natrium- oder Kaliumacetat, und gewünschtenfalls zusätzliche Leitsalze, beispielsweise Lithium-, Natrium-, Kalium-oder Tetraalkylammoniumsalze, z.B. das entsprechende Tetrafluoroborat, zugesetzt werden. Geeignete Stromdichten für die Elektrolyse liegen zwischen 10 und 400 $mA/cm^2$, beispielsweise um 40 $mA/cm^2$. Die Reaktionstemperatur liegt zwischen 0° und 50°C, vorzugsweise zwischen Raumtemperatur und 30°C.

Sowohl die oxidative Decarboxylierung mit Bleitetraacetat als auch die elektrochemische Decarboxylierung in Gegenwart eines Acetatabgebenden Mittels ergeben bevorzugt eine Verbindung der Formel I, worin die Hydroxyethylgruppe an C(3) und die Acetoxygruppe an C(4) trans zueinander angeordnet sind, also ein Diastereomeres mit (3R,4R)- oder mit (3S,4S)-Konfiguration.

Salze der genannten Verbindungen erhält man in üblicher Weise. Säureadditionssalze der Aminogruppe in Verbindungen der Formel II oder III werden beispielsweise durch Behandeln mit einer Säure oder einem geeigneten Anionenaustauscherreagens gebildet, wobei man vorzugsweise stöchiometrische Mengen oder nur einen kleinen Ueberschuss des salzbildenden Mittels verwendet. Innere Salze, z.B. von Verbindungen der Formel III, welche eine freie Carboxygruppe enthalten, können z.B. durch Neutralisieren von Salzen, wie Säureadditionssalzen, auf den isoelektrischen Punkt, z.B. mit Basen, oder durch Behandeln mit Ionenaustauschern oder Epoxiden, z.B. Propylenoxid, gebildet werden. Salze von Verbindungen der Formeln III oder IV mit Carboxygruppen kann man z.B. durch Behandeln mit Metallverbindungen, wie Alkalimetallsalzen von geeigneten organischen Carbonsäuren, z.B. dem Natriumsalz der 2-Ethylhexansäure, mit anorganischen Alkali- oder Erdalkalimetallsalzen, z.B. Natriumhydrogencarbonat, einer stöchiometrischen Menge oder einem kleinen Ueberschuss an Alkalimetallhydroxid, z.B. Lithium-, Natrium- oder Kaliumhydroxid, oder mit Ammoniak oder einem geeigneten organischen Amin bilden.

Salze können in üblicher Weise in die freien Verbindungen übergeführt werden, Metall- und Ammoniumsalze z.B. durch Behandeln mit geeigneten Säuren, Säureadditionssalze z.B. durch Behandeln mit einem geeigneten basischen Mittel.

Das Verfahren der Erfindung umfasst auch diejenigen Ausführungsformen, bei denen Zwischenprodukte isoliert und die restlichen Verfahrensschritte mit diesen durchgeführt werden, Ausgangsstoffe und Reagentien in situ hergestellt werden und/oder Zwischen- und Endprodukte ohne Isolierung weiterverarbeitet werden.

Insbesondere betrifft die Erfindung auch die im folgende genannten neuen Verfahrensschritte unter den genannten bevorzugten Reaktionsbedingungen als solche oder als Teil eines gesamten Verfahrens zur Herstellung der Verbindungen der Formel I.

Bevorzugt ist ein Verfahren zur Herstellung von Verbindungen der Formel I, dadurch gekennzeichnet, dass eine Verbindung der Formel IV oder ein Salz davon elektrochemisch in Gegenwart von Essigsäure und/oder Alkalimetallacetaten, z.B. Natrium- oder Kaliumacetat, bei Stromdichten zwischen 10 und 400 mA/cm$^2$ und Temperaturen zwischen 0° und 50°C oxidativ decarboxyliert wird.

Ganz besonders bevorzugt ist der genannte Verfahrensschritt zur Herstellung einer Verbindung der Formel Ia aus dem (3S,4S,1'R)-Diastereomeren der Verbindung der Formel IV.

Bevorzugt ist ferner ein Verfahren zur Herstellung von Verbindungen der Formel IV und von Salzen dieser Verbindungen, dadurch gekennzeichnet, dass man eine Verbindung der Formel III, worin $R^2$ Niederalkyl, $R^3$ Hydroxy, $R^4$ Wasserstoff und $Z^2$ Amino bedeuten, oder ein Salz davon mit einem Triniederalkylsilylierungsmittel, z.B. Trimethylsilylchlorid, in Gegenwart einer Base, z.B. eines Amins, z.B. Hexamethyldisilazan, in einem inerten Lösungsmittel umsetzt, die erhältliche Verbindung der Formel III, worin $R^2$ Niederalkyl, $R^3$ Triniederalkylsilyloxy, $R^4$ Triniederalkylsilyl und $Z^2$ Amino bedeuten, mit einer starken Base, beispielsweise einem nicht-nukleophilen Alkalimetallamid oder einer in 1-Stellung verzweigten Niederalkyllithium- oder Niederalkylmagnesiumverbindung, z.B. tert-Butylmagnesiumhalogenid, in einem inerten Lösungsmittel bei Temperaturen zwischen -80°C und Raumtemperatur cyclisiert und die erhältliche Verbindung hydrolysiert, und gegebenenfalls ein erhältliches Salz in die freie Verbindung oder in ein anderes Salz oder eine erhältliche freie Verbindung in ein Salz überführt.

Ganz besonders bevorzugt ist das genannte Teilverfahren zur Herstellung des (3S,4S,1'R)-Diastereomeren der Verbindung der Formel IV und von Salzen dieser Verbindung aus dem entsprechend (2S,3S,4R)-Diastereomeren der Verbindung der Formel III.

Weiter bevorzugt ist ein Verfahren zur Herstellung von Verbindungen der Formel III, worin $R^2$ Niederalkyl, $R^3$ und $R^4$ zusammen eine Bindung und $Z^2$ Amino oder Acylamino bedeuten, und von Salzen dieser Verbindunge, dadurch gekennzeichnet, dass man eine Verbindung der Formel II, worin $R^1$ Niederalkyl und $Z^1$ Amino, Arylmethylamino, Acylamino oder Azido bedeuten, oder ein Salz davon mit Wasserstoff in Gegenwart eines Edelmetallkatalysator, z.B. eines Platin-, Palladium- oder Platin/Rhodium-Katalysators, in einem inerten Lösungsmittel bei Temperaturen zwischen 0° und 100°C und einem Wasserstoffdruck zwischen 1 und 100 bar reduziert und gewünschtenfalls ein erhältliches Diastereomerengemisch mit einer Base, z.B. einem tertiären Amin oder Amidin, in einem polaren, wasserfreien Lösungsmittel bei Temperaturen zwischen 0° und 50°C in das Diastereomere mit all-trans-Anordnung der Substituenten umwandelt, und gegebenenfalls ein erhältliches Salz in die freie Verbindung oder in ein anderes Salz oder eine erhältliche freie Verbindung in ein Salz überführt.

Ganz besonders bevorzugt ist das genannte Teilverfahren zur Herstellung der (2S,3S,4R)-, (2R,3R,4S)-, (2R,3S,4S)-, (2S,3R,4R)-, (2R,3S,4R)- und (2S,3R,4S)-Diastereomeren der Verbindungen der Formel III und von Salzen dieser Verbindungen aus reinen (R)-beziehungsweise (S)-Enantiomeren der Verbindungen der Formel II.

In erster Linie betrifft die Erfindung die in den Beispielen beschriebenen Verfahren.

Weiterverarbeitung

Die Verbindung der Formel I und insbesondere das reine Diastereomere der Formel Ia kann nach einem der vielen im Stand der Technik bekannten Verfahren in wertvolle Endprodukte umgewandelt werden. Die meisten dieser Verfahren benötigen dazu ein Derivat der Verbindung der Formel I, worin die Hydroxygruppe und/oder das β-Lactam-Stick stoffatom durch eine Schutzgruppe geschützt sind. Solche Schutzgruppen lassen sich leicht einführen, beispielsweise wie in einem der oben genannten Uebersichtswerke über Schutzgruppen beschrieben.

Vorteilhafterweise wird aber die Verbindung der Formel I und ihre Diastereomeren ohne Schutzgruppen weiterverarbeitet, um zusätzliche Reaktionsschritte zu vermeiden. Beispielsweise kann die Verbindung der Formel Ia, wie in der Europäischen Patentanmeldung 215 739 beschrieben, mit der am Stickstoffatom geschützten α-Amino-thiocarbonsäure N-Allyloxycarbonyl-thioglycin umgesetzt und in wenigen Stufen zum hochwirksamen Antibiotikum (5R,6S,1'R)-2-Aminomethyl-6-(1'-hydroxyethyl)-2-penem-3-carbonsäure verarbeitet werden. Die wertvollen Eigenschaften des genannten Endprodukts und seine Verwendung sind beispielsweise in der Deutschen Patentanmeldung DE 34 31 980 beschrieben.

Zwischenprodukte

Neue Zwischenprodukte sind ebenfalls Gegenstand der Erfindung.

Insbesondere betrifft die Erfindung Verbindungen der Formel III, worin $R^2$ Wasserstoff, Niederalkyl, z.B. Methyl oder Ethyl, oder eine Carboxyschutzgruppe, beispielsweise Triniederalkylsilyl, z.B. Trimethylsilyl, tert-Butyldimethylsilyl oder 1,1,2-Trimethylpropyldimethyl-silyl, arylsubstituiertes Silyl, z.B. Dimethylphenylsilyl oder Triphenylsilyl, in 1-Stellung verzweigtes und/oder substituiertes Niederalkyl, z.B. tert-Butyl, Diphenylmethyl, Di(p-methoxyphenyl)methyl, Trityl, Methoxymethyl, 1-Ethoxyethyl oder Methylthiomethyl, oder 2-Oxa- oder 2-Thiacycloalkyl mit 5-7 Ringatomen, z.B. 2-Tetrahydrofuryl oder 2-Tetrahydropyranyl, $R^3$ Hydroxy oder geschütztes Hydroxy, beispielsweise Triniederalkylsilyloxy, z.B. Trimethylsilyloxy , tert-Butyldimethylsilyloxy oder 1,1,2-Trimethylpropyl-dimethyl-silyloxy, arylsubstituiertes Silyloxy, z.B. Dimethylphenylsilyloxy oder

Triphenylsilyloxy, in 1-Stellung verzweigtes und/oder substituiertes Niederalkoxy, z.B. tert-Butoxy, Diphenylmethoxy, Di(p-methoxyphenyl)methoxy, Trityloxy, Methoxy methoxy, 1-Ethoxyethoxy oder Methylthiomethoxy, oder 2-Oxa- oder 2-Thiacycloalkoxy mit 5-7 Ringatomen, z.B. 2-Tetrahydrofuryloxy oder 2-Tetrahydropyranyloxy, $R^4$ Wasserstoff oder eine Carboxyschutzgruppe, beispielsweise eine unter $R^2$ genannte Carboxyschutzgruppe, oder $R^3$ und $R^4$ zusammen eine Bindung, und $Z^2$ Amino oder Acylamino, beispielsweise unsubstituiertes oder substituiertes Niederalkanoylamino, z.B. Formylamino, Acetylamino, Propionylamino, tert-Butylcarbonylamino, Chloracetylamino, Trichloracetylamino oder Trifluoracetylamino, Niederalkoxycarbonylamino, z.B. tert-Butoxycarbonylamino, Arylniederalkoxycarbonylamino, z.B. Benzyloxycarbonylamino, 4-Nitrobenzyloxycarbonylamino oder Diphenylmethoxycarbonylamino, oder Arylcarbonylamino, z.B. Benzoylamino, bedeuten, deren reine Diastereomere und Salze davon.

Besonders betrifft die Erfindung reine Diastereomere der Verbindungen der Formel III, worin $R^2$ Wasserstoff, Niederalkyl, z.B. Methyl oder Ethyl, oder eine Carboxyschutzgruppe, beispielsweise Triniederalkylsilyl, z.B. Trimethylsilyl, $R^3$ Hydroxy oder geschütztes Hydroxy, beispielsweise Triniederalkylsilyloxy, z.B. Trimethylsilyloxy, $R^4$ Wasserstoff oder eine Carboxyschutzgruppe, beispielsweise Triniederalkylsilyl, z.B. Trimethylsilyl, oder $R^3$ und $R^4$ zusammen eine Bindung, und $Z^2$ Amino, Niederalkanoylamino, z.B. Acetylamino oder tert-Butylcarbonylamino, Niederalkoxycarbonylamino, z.B. tert-Butoxycarbonylamino, oder Arylcarbonylamino, z.B. Benzoylamino, bedeuten, und Salze davon.

Hauptsächlich betrifft die Erfindung die (2S,3S,4R)-, (2R,3R,4S)-, (2R,3S,4S)-, (2S,3R,4R)-, (2R,3S,4R)- und (2S,3R,4S)-Diastereomeren der Verbindungen der Formel III, worin $R^2$ Wasserstoff, Niederalkyl, z.B. Methyl oder Ethyl, Triniederalkylsilyl, z.B. Trimethylsilyl, $R^3$ Hydroxy oder Triniederalkylsilyloxy, z.B. Trimethylsilyloxy, $R^4$ Wasserstoff oder Triniederalkylsilyl, z.B. Trimethylsilyl, oder $R^3$ und $R^4$ zusammen eine Bindung, und $Z^2$ Amino oder Niederalkanoylamino, z.B. Acetylamino oder tert-Butylcarbonylamino, bedeuten, und Salze davon.

In erster Linie betrifft die Erfindung die (2S,3S,4R)-, (2R,3R,4S)-, (2R,3S,4S)- und (2R,3S,4R)-Diastereomeren der Verbindungen der Formel III, worin $R^2$ Wasserstoff oder Niederalkyl, z.B. Methyl oder Ethyl, $R^3$ und $R^4$ zusammen eine Bindung und $Z^2$ Amino oder Niederalkanoylamino, z.B. Acetylamino, bedeuten, und Salze davon.

Gleichermassen betrifft die Erfindung die (2S,3S,4R)-Diastereomeren der Verbindungen der Formel III, worin $R^2$ Wasserstoff oder Niederalkyl, z.B. Methyl oder Ethyl, $R^3$ Hydroxy oder Triniederalkylsilyloxy, z.B. Trimethylsilyloxy, $R^4$ Wasserstoff oder Triniederalkylsilyl, z.B. Trimethylsilyl, und $Z^2$ Amino bedeuten, und Salze davon.

Weiter betrifft die Erfindung die Verbindung der Formel IV und deren reine Diastereomere, insbesondere das (3S,4S,1'R)-Diastereomer.

In erster Linie betrifft die Erfindung die in den Beispielen genannten Zwischenprodukte.

Ausgangsmaterialien

Neue Ausgangsmaterialien und Verfahren zu deren Herstellung sind ebenfalls Gegenstand der Erfindung. Insbesondere betrifft die Erfindung reine Enantiomere der Verbindungen der Formel II, worin $R^1$ Niederalkyl und $Z^1$ Hydroxy, verethertes Hydroxy, verestertes Hydroxy, Amino, Arylmethylamino, Acylamino oder Azido bedeuten, tautomere Formen dieser Verbindungen, beispielsweise die Verbindungen der Formel IIa, worin $R^1$ Niederalkyl und Y Oxo, Imino, Arylmethylimino oder Acylimino bedeuten, und Salze dieser Verbindungen mit salzbildenden Gruppen.

Verethertes Hydroxy $Z^1$ ist beispielsweise unsubstituiertes oder substituiertes Niederalkoxy, beispielsweise Niederalkoxy, z.B. Methoxy oder Ethoxy, Niederalkoxyniederalkoxy, z.B. 1- oder 2-Methoxy-oder Ethoxy-ethoxy oder Methoxymethoxy, Niederalkanoyloxyniederalkoxy, z.B. Acetoxymethoxy oder tert-Butylcarbonyloxymethoxy, oder Arylniederalkoxy, z.B. Benzyloxy oder 2-Phenylethoxy, Aryloxy, beispielsweise Phenyloxy, oder Silyloxy, beispielsweise Triniederalkylsilyloxy, z.B. Trimethylsilyloxy oder tert-Butyldimethylsilyloxy, oder Triarylsilyloxy, z.B. Triphenylsilyloxy.

Verestertes Hydroxy $Z^1$ ist beispielsweise mit einer Mineralsäure, einer organischen Sulfonsäure oder einer Carbonsäure verestert und ist beispielsweise Halogen, z.B. Chlor, Brom oder Iod, unsubstituiertes oder substituiertes Niederalkansulfonyloxy, z.B. Methansulfonyloxy, Trifluormethansulfonyloxy oder Campher-10-sulfonyloxy, Arylsulfonyloxy, z.B. Benzolsulfonyloxy, p-Toluolsulfonyloxy, p-Nitrobenzolsulfonyloxy oder 2,4-Dinitrobenzolsulfonyloxy, unsubstituiertes oder substituiertes Niederalkanoyloxy, z.B. Formyloxy, Acetoxy, Propionoxy, tert-Butylcarbonyloxy, Chloracetoxy, Fluoracetoxy oder Trifluoracetoxy, Niederalkoxycarbonyloxy, z.B. Methoxycarbonyloxy oder n- oder tert-Butoxycarbonyloxy, oder Arylcarbonyloxy, z.B. Benzoyloxy.

Besonders betrifft die Erfindung reine Enantiomere der Verbindungen der Formel II, worin $R^1$ Niederalkyl, z.B. Methyl, Ethyl oder tert-Butyl, und $Z^1$ Hydroxy, unsubstituiertes oder substituiertes Niederalkoxy, beispielsweise unsubstituiertes Niederalkoxy, z.B. Methoxy oder Ethoxy, Niederalkoxyniederalkoxy, z.B. 1- oder 2-Methoxy- oder Ethoxy-ethoxy oder Methoxymethoxy, Niederalkanoyloxyniederalkoxy, z.B. Acetoxymethoxy oder tert-Butylcarbonyloxymethoxy, oder Arylniederalkoxy, z.B. Benzyloxy oder 2-Phenylethoxy, Aryloxy, beispielsweise Phenyloxy, Silyloxy, beispielsweise Triniederalkylsilyloxy, z.B. Trimethylsilyloxy oder tert-Butyldimethylsilyloxy, oder Triarylsilyloxy, z.B. Triphenylsilyloxy, Halogen, z.B. Chlor, Brom oder Iod, unsubstituiertes oder substituiertes Niederalkansulfonyloxy, z.B. Methansulfonyloxy, Trifluormethansulfonyloxy oder Campher-10-sulfonyloxy, Arylsulfonyloxy, z.B. Benzolsulfonyloxy, p-Toluolsulfonyloxy, p-Nitrobenzolsulfonyloxy oder 2,4-Dinitrobenzolsulfonyloxy, unsubstituiertes oder substituiertes Niederalkanoyloxy, z.B. Formyloxy, Acetoxy, Propionoxy, tert-Butylcarbonyloxy, Chloracetoxy, Fluoracetoxy oder Trifluoracetoxy,

Niederalkoxycarbonyloxy, z.B. Methoxycarbonyloxy oder n- oder tert-Butoxycarbonyloxy, Arylcarbonyloxy, z.B. Benzoyloxy, Amino, Arylmethylamino, z.B. Benzylamino, Dibenzylamino, 2-, 3- oder 4-Methylbenzylamino, α- oder β-Naphthylamino oder Diphenylmethylamino, unsubstituiertes oder substituiertes Niederalkanoylamino, z.B. Formylamino, Acetylamino, Propionylamino, tert-Butylcarbonylamino, Chloracetylamino, Trichloracetylamino oder Trifluoracetylamino, Niederalkoxycarbonylamino, z.B. tert-Butoxycarbonylamino, Arylniederalkoxycarbonylamino, z.B. Benzyloxycarbonylamino, 4-Nitrobenzyloxycarbonylamino oder Diphenylmethoxycarbonylamino, Arylcarbonylamino, z.B. Benzoylamino, oder Azido bedeuten, tautomere Formen dieser Verbindungen, beispielsweise die Verbindungen der Formel IIa, worin $R^1$ Niederalkyl und Y Oxo, Imino, Arylmethylimino oder Acylimino bedeuten, und Salze dieser Verbindungen mit salzbildenden Gruppen.

Hauptsächlich betrifft die Erfindung reine Enantiomere der Verbindungen der Formel II, worin $R^1$ Niederalkyl, z.B. Methyl, Ethyl oder tert-Butyl, und $Z^1$ Hydroxy, Halogen, z.B. Chlor, Brom oder Iod, unsubstituiertes oder substituiertes Niederalkansulfonyloxy, z.B. Methansulfonyloxy, Trifluormethansulfonyloxy oder Campher-10-sulfonyloxy, Arylsulfonyloxy, z.B. Benzolsulfonyloxy, p-Toluolsulfonyloxy, p-Nitrobenzolsulfonyloxy oder 2,4-Dinitrobenzolsulfonyloxy, Amino, Arylmethylamino, z.B. Benzylamino oder Dibenzylamino, Niederalkanoylamino, z.B. Acetylamino oder tert-Butylcarbonylamino, Niederalkoxycarbonylamino, z.B. tert-Butoxycarbonylamino, Arylcarbonylamino, z.B. Benzoylamino, oder Azido bedeuten, tautomere Formen dieser Verbindungen, beispielsweise die Verbindungen der Formel IIa, worin $R^1$ Niederalkyl und Y Oxo, Imino, Arylmethylimino oder Acylimino bedeuten, und Salze dieser Verbindungen mit salzbildenden Gruppen.

In erster Linie betrifft die Erfindung reine Enantiomere der Verbindungen der Formel II, worin $R^1$ Niederalkyl, z.B. Methyl, Ethyl oder tert-Butyl, und $Z^1$ Hydroxy, Niederalkansulfonyloxy, z.B. Methansulfonyloxy, Camphersulfonyloxy, z.B. d-Campher-10-sulfonyloxy, Arylsulfonyloxy, z.B. p-Toluolsulfonyloxy, p-Nitrobenzolsulfo nyloxy oder 2,4-Dinitrobenzolsulfonyloxy, Amino, Arylmethylamino, z.B. Benzylamino, Niederalkanoylamino, z.B. Acetylamino, oder Azido bedeuten, tautomere Formen dieser Verbindungen, beispielsweise die Verbindungen der Formel IIa, worin $R^1$ Niederalkyl und Y Oxo, Imino, Arylmethylimino oder Niederalkanoylimino bedeuten, und Salze dieser Verbindungen mit salzbildenden Gruppen.

Die neuen erfindungsgemässen Verbindungen der Formel II und ihre Salze werden nach an sich bekannten Verfahren hergestellt, beispielsweise indem man

a) eine enantiomerenreine Verbindung der Formel

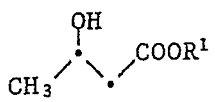

(V),

worin $R^1$ Niederalkyl bedeutet, mit einem Oxalsäurediester kondensiert, oder

b) eine racemische Verbindung der Formel II, worin $R^1$ Niederalkyl und $Z^1$ Hydroxy, Amino oder Arylmethylamino bedeuten, mit einer enantiomerenreinen, chiralen Säure oder einem reaktiven Derivat davon umsetzt, das erhältliche Diastereomerengemisch in die Diastereomeren auftrennt und, wenn erwünscht, die reinen Diastereomeren rückspaltet, oder

c) in einer prochiralen Verbindung der Formel

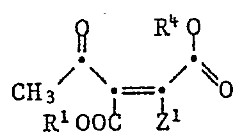

(VI),

worin $R^1$ Niederalkyl und $R^4$ Wasserstoff oder eine Carboxyschutzgruppe bedeuten und $Z^1$ die unter Formel II genannten Bedeutungen hat, mit einem chiralen Reduktionsmittel die Carbonylgruppe reduziert und, gegebenenfalls nach Abspaltung der Schutzgruppe $R^4$, mit Säure den Lactonring schliesst, und gegebenenfalls eine erhältliche enantiomerenreine Verbindung der Formel II in eine andere enantiomerenreine Verbindung der Formel II umwandelt und/oder ein erhältliches Salz in die freie Verbindung oder in ein anderes Salz oder eine erhältliche freie Verbindung in ein Salz überführt.

Die Kondensation in Teilverfahren a) wird unter den üblichen Reaktionsbedingungen einer gekreuzten Esterkondensation durchgeführt. Der Hydroxybuttersäureester wird mit zwei oder mehr Aequivalenten einer starken, nicht-nukleophilen Base deprotoniert und das gebildete Dianion mit dem Oxalsäureester in einem polaren, vorzugsweise aprotischen Lösungsmittel umgesetzt.

Geeignete nicht-nukleophile Basen sind beispielsweise Alkalimetallamide, z.B. Lithiumdiisopropylamid, Lithiumcyclohexylethylamid, Lithium-2,2,6,6-tetramethylpiperidid oder Lithium-, Natrium- oder Kalium-bis(trimethylsilyl)amid, verzweigte Alkyllithiumverbindungen, z.B. tert-Butyllithium, oder Alkalimetallhydride, z.B. Natrium- oder Kaliumhydrid.

Geeignete polare, aprotische Lösungsmittel sind beispielsweise Ether, z.B. Tetrahydrofuran oder Dimethoxyethan, Amide, z.B. Dimethylformamid, N-Methylpyrrolidon oder Hexamethylphosphorsäuretriamid, Dimethylsulfoxid, cyclische Harnstoffe, z.B. N,N'-Dimethyl-N,N'-propylenharnstoff, ferner Mischungen der genannten Lösungsmittel untereinander und/oder mit Kohlenwasserstoffen, z.B. n-Pentan, n-Hexan oder

Cyclohexan. Als Lösungsmittel können auch Niederalkanole verwendet werden, beispielsweise der Alkohol $R^1OH$, z.B. Methanol, Ethanol oder tert-Butanol, wobei dann als Basen die entsprechenden Alkalimetallalkoholate eingesetzt werden, z.B. Natriumethanolat oder Kalium-tert-butanolat.

Die Kondensationsreaktion wird vorzugsweise bei tiefen Temperaturen durchgeführt, z.B. zwischen -100° und 0° C, beispielsweise zwischen -78° und -30° C, wenn erwünscht, unter einer Schutzgasatmosphäre, z.B. Stickstoff oder Argon.

In Teilverfahren b) wird beispielsweise eine racemische Verbindung der Formel II, worin $Z^1$ Amino oder Arylmethylamino, z.B. Benzylamino, bedeutet, mit einer enantiomerenreinen, chiralen Säure in ein Gemisch zweier diastereomerer Säureadditionssalze übergeführt. Geeignete Säuren sind z.B. chirale Carbonsäuren, z.B. Weinsäure, Diacetylweinsäure, Aepfelsäure, Glutaminsäure, Pyrrolidon-5-carbonsäure (Pyroglutaminsäure) oder Menthoxyessigsäure, ferner chirale Sulfonsäuren, z.B. Campher-10-sulfonsäure. Die erhältlichen diastereomeren Salze werden mit üblichen physikalischen Methoden getrennt, beispielsweise durch fraktionierte Kristallisation. Die diastereomerenreinen Salze können auf übliche Weise in die enantiomerenreinen Verbindungen der Formel II rückgespalten werden, beispielsweise durch Behandlung mit Base oder mit einem Ionenaustauscher.

Racemische Verbindungen der Formel II, worin $Z^1$ Hydroxy bedeutet, werden mit einer enantiomerenreinen, chiralen Säure oder einem geeigneten Derivat dieser Säuren verestert. Die Veresterung mit einer der genannten chiralen Carbonsäuren selbst erfolgt unter den üblichen Bedingungen, beispielsweise in Gegenwart einer wasserfreien Mineralsäure, einer organischen Sulfonsäure oder einer Lewis-Säure, wie weiter vorn bei der Umwandlung von Verbindungen der Formel III ineinander beschrieben. Vorzugsweise werden für die Versterung aber Derivate der genannten chiralen Carbon- oder Sulfonsäuren eingesetzt, beispielsweise Anhydride, z.B. symmetrische Carbonsäureanhydride, oder Halogenide, z.B. Säurechloride. Die Veresterung mit einem Säurechlorid, z.B. Pyroglutaminsäurechlorid, Menthoxyessigsäurechlorid oder insbesondere Campher-10-sulfonsäurechlorid erfolgt in einem inerten Lösungsmittel, z.B. Toluol, Diethylether oder Methylenchlorid, in Gegenwart einer Base, z.B. eines tertiären Amins, z.B. Triethylamin oder N-Methylmorpholin, oder eines Alkali-oder Erdalkalicarbonats, z.B. Natrium-, Kalium- oder Calciumcarbonat, bei Temperaturen zwischen -30° C und 50° C, vorzugsweise um 0° C. Die erhältlichen diastereomeren Ester werden mit den üblichen physikalischen Methoden getrennt, beispielsweise durch fraktionierte Kristallisation oder durch Säulenchromatographie. Die erhältlichen diastereomerenreinen Ester sind enantiomerenreine Verbindungen der Formel II, worin $Z^1$ chiral verestertes Hydroxy bedeutet. Wenn erwünscht, können sie nach den üblichen Methoden der Esterhydrolyse in enantiomerenreine Verbindungen der Formel II, worin $Z^1$ Hydroxy bedeutet, rückgespalten werden.

Racemische Verbindungen der Formel II, worin $Z^2$ Hydroxy bedeutet, können auch mit einem Anhydrid einer Disäure verestert werden, z.B. mit Phthalsäure-, Bernsteinsäure- oder Maleinsäureanhydrid, und die erhältlichen Säuren durch Salzbildung mit enantiomerenreinen, chiralen Aminen z.B. α-Phenethylamin, Brucin oder Abietylamin, in Gemische diastereomerer Salze, die sich durch Kristallisation trennen lassen, übergeführt werden. Die Rückspaltung nach den üblichen Methoden ergibt enantiomerenreine Verbindungen der Formel II.

Racemische Verbindungen der Formel II, worin $Z^1$ Amino oder Arylmethylamino bedeutet, können in der weiter vorn beschriebenen Art und Weise an der Aminogruppe acyliert werden. Verwendet man als Acylierungsmittel Derivate enantiomerenreiner, chiraler Säuren, z.B. der obengenannten Carbon- oder Sulfonsäuren, so werden Gemische diastereomerer Amide gebildet, die sich durch fraktionierte Kristallisation oder durch Chromatographie trennen lassen. Die erhältlichen diastereomerenreinen Amide sind enantiomerenreine Verbindungen der Formel II, worin $Z^1$ chirales Acylamino bedeutet.

Geeignete chirale Reduktionsmittel für Teilverfahren c) sind beispielsweise enantiomerenreine, chirale Borane, die ein übertragbares Wasserstoffatom enthalten, z.B. (+)- oder (-)-Diisopinocampheylboran, oder Lithiumaluminiumhydrid in Gegenwart von enantiomerenreinen, chiralen Alkoholen, Aminoalkoholen oder Aminen, z.B. 1,1'-Di(β-naphthol), Darvonalkohol oder 2,6-Dimethyl-N-(2-pyrrolidinylmethyl)-anilin. Geeignete Lösungsmittel sind die in Hydroborierungen beziehungsweise Hydrid-Reduktionen üblichen Lösungsmittel, z.B. wasserfreie Ether, z.B. Diethylether, Tetrahydrofuran oder 1,2-Dimethoxyethan. Vorzugsweise werden die Reduktionen bei tiefen Temperaturen durchgeführt, z.B. zwischen -100° C und 0° C, z.B. um -30° C.

Die bei der Reduktion erhältliche Hydroxyverbindung wird, gegebenenfalls nach Abspaltung der Schutzgruppe $R^4$ in der weiter vorn beschrieben Art und Weise, mit Säure zum Lacton der Formel II zyklisiert. Geeignete Säuren und Reaktionsbedingungen sind weiter vorn unter der Veresterung von Verbindungen der Formel III genannt, wobei jedoch zur Lactonringbildung der Alkohol der Formel $R^2OH$ weggelassen wird.

Verbindungen der Formel II, worin $Z^1$ Hydroxy, verethertes Hydroxy oder verestertes Hydroxy bedeutet, werden nach an sich bekannten Methoden in Verbindungen der Formel II umgewandelt, worin $Z^1$ Amino, Arylmethylamino, Acylamino oder Azido bedeutet. Beispielsweise wird die Hydroxygruppe in eine reaktive Abgangsgruppe, z.B. ein Halogenid, z.B. Chlorid, oder ein Sulfonat, z.B. ein Niederalkansulfonat, z.B. Mesylat, oder ein Arylsulfonat, z.B. Benzolsulfonat p-Toluolsulfonat, p-Nitrobenzolsulfonat oder 2,4-Dinitrobenzol-umgewandelt und diese reaktive Abgangsgruppe durch ein Arylmethylamin, z.B. Benzylamin oder Dibenzylamin, oder durch Azid substituiert. Verbindungen der Formel II, worin $Z^1$ verethertes oder verestertes Hydroxy bedeutet, werden vorgängig nach üblichen Methoden der Ether- bzw. Esterspaltung, z.B. mit Säure oder Base, in eine Verbindung umgewandelt, worin $Z^1$ Hydroxy bedeutet, es sei denn, die veretherte oder veresterte Hydroxygruppe sei selbst eine reaktive Abgangsgruppe, die sich durch Azid oder ein

Arylmethylamin substituieren lässt, z.B. 2,4-Dinitrophenyloxy, Halogen, z.B. Chlor, Brom oder Iod, Niederalkansulfonyloxy, z.B. Mesyloxy oder Campher-10-sulfonyloxy, oder Arylsulfonyloxy, z.B. Benzolsulfonyloxy, p-Toluolsulfonyloxy, p-Nitrobenzolsulfonyloxy oder 2,4-Dinitrobenzolsulfonyloxy.

die Umwandlung von Hydroxy in Halogen erfolgt mit den üblichen Halogenierungsmitteln, beispielsweise Phosphortri- oder -pentahalogenid, z.B. Phosphortri- oder -pentachlorid, Phosphortribromid oder Phosphoroxychlorid, Triphenylphosphordihalogenid, z.B. Triphenylphosphordichlorid, oder Thionylchlorid, in einem inerten Lösungsmittel, beispielsweise Diethylether, Toluol, Dioxan oder Dimethylformamid, oder in einem Ueberschuss des Reagens, gegebenenfalls in Gegenwart einer Base, z.B. Pyridin, bei Temperaturen zwischen -30° C und 50° C, z.B. zwischen 0° C und Raumtemperatur. Die Umwandlung von Hydroxy in ein Sulfonat erfolgt vorzugsweise mit dem entsprechenden Sulfonsäurehalogenid, z.B. Sulfonsäurechlorid, in einem inerten Lösungsmittel, z.B. Toluol, Diethylether oder Methylenchlorid, in Gegenwart einer Base, z.B. eines tertiären Amins, z.B. Triethylamin oder N-Methylmorpholin, oder eines Alkali-oder Erdalkalicarbonats, z.B. Natrium-, Kalium- oder Calciumcarbonat, bei Temperaturen zwischen -30° C und 50° C, vorzugsweise um 0° C.

Verbindungen der Formel II, in denen $Z^1$ eine der obengenannten Abgangsgruppen bedeutet, werden dann mit einem Arylmethylamin, z.B. Benzylamin, Dibenzylamin, 2-, 3- oder 4-Methylbenzylamin, α- oder β-Naphthylmethylamin oder Diphenylmethylamin, oder mit einem Alkaliazid, z.B. Natrium- oder Kaliumazid, in einem polaren Lösungsmittel, z.B. Wasser, Alkohol, z.B. Methanol oder Ethanol, Essigsäure, Aceton, Acetonitril, Dimethylformamid oder Dimethylsulfoxid oder Mischungen derselben, bei Temperaturen zwischen 0° und 50° C, vorzugsweise um Raumtemperatur, umgesetzt. Die Reaktion mit Arylmethylamin kann auch ohne Lösungsmittel in einem Ueberschuss des Reagens oder in wenig polaren Lösungsmitteln, z.B. Methylenchlorid oder Toluol, erfolgen.

Enantiomerenreine Verbindungen der Formel V, z.B. (S)- oder (R)-3-Hydroxybuttersäureester, sind bekannt oder können nach bekannten Methoden hergestellt werden, z.B. durch Veresterung der durch Depolymerisation von Polyhydroxybuttersäure zugänglichen Hydroxysäure.

Racemische Verbindungen der Formel II sind bekannt oder können nach einer der vorstehend beschriebenen Methoden für die Umwandlung von Verbindungen der Formel II oder III aus der bekannten racemischen Verbindung der Formel II, worin $R^1$ Ethyl und $Z^1$ Hydroxy bedeutet, hergestellt werden.

Verbindungen der Formel VI sind bekannt oder können nach bekannten Methoden hergestellt werden, beispielsweise durch gekreuzte Esterkondensation von Acetessigsäureester mit Oxalsäureester oder von Essigsäureester mit Oxalessigsäureester analog dem in Teilverfahren a) beschriebenen Verfahren.

Die folgenden Beispiele dienen zur Illustration der Erfindung, schränken deren Umfang jedoch in keiner Weise ein.

Die Werte für Proton-Kernresonanzspektroskopie ($^1$H-NMR) werden in ppm (parts per million) bezogen auf Tetramethylsilan (δ = 0) als internen Standard angegeben. s = Singulett, d = Dublett, t = Triplett, q = Quartett, m = Multiplett, dxd = Doppeldublett. Optische Drehungen $[\alpha]_D$ werden, wo nicht anders angegeben, bei der Natrium-D-Linie gemessen, Konzentration in g pro 100 ml.

Beispiel 1: (R)-2-Hydroxy-3-methoxycarbonyl-2-penten-4-olid

Zu einer Lösung von 1,27 Mol Diisopropylamin in 600 ml Tetrahydrofuran wird bei -25° C innerhalb von 20 Minuten 775 ml 1,6 M n-Butyllithiumlösung in n-Hexan zugetropft. Anschliessend werden bei -70° C 70,9 g (R)-3-Hydroxybuttersäuremethylester innerhalb von 30 Minuten und nach 1 Stunde 70,9 g Oxalsäuredimethylester innerhalb von 40 Minuten zugetropft. Nach zwei Stunden Nachreaktionszeit bei -60° C wird auf 0° C erwärmt und mit 430 ml 6 N Salzsäure auf pH 2 angesäuert. Das Produkt wird durch mehrfache Extraktion mit Essigester isoliert und durch Extraktion in wässrige Bicarbonatlösung, Ansäuern mit 6 N Salzsäure auf pH 2,5 und nochmalige Extraktion mit Essig ester vorgereinigt. Das Lösungsmittel wird abdestilliert und der Rückstand aus Ether/Petrolether umkristallisiert. $[\alpha]^{20}_D$ = +30,0° (3 % in $CHCl_3$), Smp. 94-96° C.

Beispiel 2:

Analog Beispiel 1 werden hergestellt:

a) (R)-2-Hydroxy-3-ethoxycarbonyl-2-penten-4-olid, $[\alpha]^{20}_D$ = +25,0° (3 % in $CHCl_3$), Smp. 56-57° C, aus (R)-3-Hydroxybuttersäureethylester.

b) (S)-2-Hydroxy-3-methoxycarbonyl-2-penten-4-olid, $[\alpha]^{20}_D$ = -30° (3 % in $CHCl_3$), Smp. 94-96° C, aus (S)-3-Hydroxybuttersäuremethylester.

Beispiel 3: (R)- und (S)-2-(d-Campher-10-sulfonyloxy)-3-ethoxycarbonyl-2-penten-4-olid

52,1 g racemisches 2-Hydroxy-3-ethoxycarbonyl-2-penten-4-olid (hergestellt nach A. Rossi und H. Schinz, Helv. Chim. Acta 31, 473 (1948)) und 87,8 g d(+)-Campher-10-sulfonylchlorid werden in 280 ml Toluol gelöst und bei -10° C mit 33,4 g Triethylamin umgesetzt. Die Lösung wird angesäuert, neutral gewaschen, über Kieselgel filtriert und eingedampft. Das orangerote Oel wird aus Essigester/Cyclohexan fraktioniert kristallisiert. Dabei kristallisiert zunächst das (S)/d-Camphersulfonylderivat aus, während sich das (R)/d-Camphersulfonylderivat in der Mutterlauge anreichert. Durch wiederholte Fraktionierung wird auch das (R)/d-Camphersulfonylderivat kristallin erhalten.

(S)/d-Camphersulfonylderivat: $[\alpha]^{20}_D$ = -11° (3 % in $CHCl_3$), Smp. 114-115° C, IR 1765, 1730, 1710 $cm^{-1}$.
(R)/d-Camphersulfonylderivat: $[\alpha]^{20}_D$ = +16° (3 % in $CHCl_3$), Smp. 75-77° C, IR 1765, 1730, 1710 $cm^{-1}$.

Beispiel 4: (R)-2-Mesyloxy-3-methoxycarbonyl-2-penten-4-olid

137,76 g (R)-2-Hydroxy-3-methoxycarbonyl-2-penten-4-olid werden in 660 ml Toluol gelöst und bei -10°C mit 110 g Methansulfonylchlorid und anschliessend innerhalb von 1 Stunde mit 97 g Triethylamin versetzt. Es werden 350 ml Wasser und 150 ml gesättigte Kochsalzlösung zugegeben, die toluolische Produktphase abgetrennt und die wässrige Phase zweimal mit 250 ml Toluol nachextrahiert. Die Toluolphasen werden mit Kochsalzlösung und mit Wasser gewaschen und eingedampft. Orangerotes Oel, $[\alpha]^{20}_{D}$ = +10,6° (3 % in $CHCl_3$).

Beispiel 5: (R)-2-(2,4-Dinitrobenzolsulfonyloxy)-3-ethoxycarbonyl-2-penten-4-olid

Die Titelverbindung wird analog Beispiel 4 aus 2,4-Dinitrobenzolsulfonylchlorid und (R)-2-Hydroxy-3-ethoxycarbonyl-2-penten-4-olid hergestellt, $[\alpha]^{20}_{D}$ = -17° (3 % in $CHCl_3$), Smp. 95-96°C.

Beispiel 6: (R)-2-(4-Nitrobenzolsulfonyloxy)-3-methoxycarbonyl-2-penten-4-olid

34,44 g (R)-2-Hydroxy-3-methoxycarbonyl-2-penten-4-olid werden in 170 ml Toluol bei 40°C gelöst, mit 49,4 g p-Nitrobenzolsulfonylchlorid in 160 ml Toluol versetzt und auf 5°C abgekühlt. Innerhalb von 30 Minuten werden bei 0° bis 5°C 20,3 g N-Methylmorpholin zudosiert. Es werden 350 ml Wasser zugegeben, die toluolische Produktphase abgetrennt und die wässrige Phase zweimal mit 100 ml Toluol nachextrahiert. Die Toluolphasen werden mit Kochsalzlösung und mit Wasser gewaschen, über wenig Kieselgel filtriert und eingedampft. Der Rückstand wird aus Essigester/Cyclohexan kristallisiert. $[\alpha]^{20}_{D}$ = +12,4° (3 % in $CHCl_3$), Smp. 123-124°C.

Beispiel 7: (R)-2-Benzylamino-3-methoxycarbonyl-2-penten-4-olid

Zu einer Lösung von 35,7 g (R)-2-(4-Nitrobenzolsulfonyloxy)-3-methoxycarbonyl-2-penten-4-olid in 250 ml Methylenchlorid werden bei 0°C innerhalb von 1 Stunde 25,5 g Benzylamin zugetropft. Das ausgefallene Benzylamin-p-nitrobenzolsulfonsäuresalz wird abfiltriert, das Filtrat eingedampft, mit Toluol über Kieselgel filtriert und aus Ether/Petrolether umkristallisiert. $[\alpha]^{20}_{D}$ = +22° (3 % in $CHCl_3$), Smp. 68-70°C, IR ($CH_2Cl_2$) 1752, 1678, 1632 $cm^{-1}$.

Beispiel 8: (R)-2-Azido-3-methoxycarbonyl-2-penten-4-olid

Zu einer Lösung von 190,4 g (R)-2-Mesyloxy-3-methoxycarbonyl-2-penten-4-olid in 275 ml Aceton und 55 ml Eisessig wird bei 20°C eine Lösung von 62 g Natriumazid in 180 ml Wasser innerhalb 1 Stunde so zugetropft, dass die Innentemperatur nicht über 25°C steigt. Nach 4 Stunden bei 25°C wird mit 500 ml Wasser und 500 ml Toluol verrührt. Die Toluolphase wird abgetrennt, die wässrige Phase am Rotationsverdampfer von Aceton befreit und mehrmals mit Toluol extrahiert. Die Toluolextrakte werden getrocknet und im Vakuum zu einer ca. 30%igen Lösung aufkonzentriert. Eine Probe wird eingedampft: Oel, $[\alpha]^{20}_{D}$ = +8° (3 % in $CH_3CN$), IR 2110 $cm^{-1}$.

Die Titelverbindung lässt sich analog auch aus den entsprechenden (R)-2-(2,4-Dinitrobenzolsulfonyloxy)-, 2-(4-Nitrobenzolsulfonyloxy)-oder 2-(d-Campher-10-sulfonyloxy)-3-methoxycarbonyl-2-penten-4-oliden herstellen.

Beispiel 9: (R)-2-Amino-3-methoxycarbonyl-2-penten-4-olid durch Reduktion des Azids

Die ca. 30%ige toluolische Lösung enthaltend 0,75 Mol (R)-2-Azido-3-methoxycarbonyl-2-penten-4-olid von Beispiel 8 wird mit 1,0 l Essigester verdünnt, mit 22 g Pd/C (5 %) versetzt und bei 20-25°C bei Normaldruck 3-4 Stunden hydriert. Der Katalysator wird abfiltriert, das Lösungsmittel abdestilliert und der Rückstand aus Essigester/n-Heptan umkristallisiert, $[\alpha]^{20}_{D}$ = +30,9° (3 % in $CHCl_3$), Smp. 106-108°C.

Beispiel 10:

Analog Beispiel 8 und 9 werden ausgehend von den Camphersulfonaten von Beispiel 3 über das entsprechende Azid hergestellt:

a) (R)-2-Amino-3-ethoxycarbonyl-2-penten-4-olid, $[\alpha]^{20}_{D}$ = +25,1°.
b) (S)-2-Amino-3-ethoxycarbonyl-2-penten-4-olid, $[\alpha]^{20}_{D}$ = -24,5°.

Beispiel 11: (R)-2-Amino-3-methoxycarbonyl-2-penten-4-olid durch Hydrierung des Benzylamins

3 g (R)-2-Benzylamino-3-methoxycarbonyl-2-penten-4-olid werden in 30 ml Ethanol gelöst, mit 0,3 g Pd/C (5 %) versetzt, mit 4 ml 10%-iger ethanolischer Salzsäurelösung verdünnt und bei 35°C und Normaldruck bis zur Sättigung hydriert. Der Katalysator wird abfiltriert, das Lösungsmittel abdestilliert und der Rückstand aus Essigester/Cyclohexan umkristallisiert. $[\alpha]^{20}_{D}$ = +31° (3 % in $CHCl_3$), Smp. 106-108°C.

Beispiel 12: (R)-2-Acetamido-3-ethoxycarbonyl-2-penten-4-olid

12,04 g (R)-2-Amino-3-ethoxycarbonyl-2-penten-4-olid werden in 48 ml Acetanhydrid gelöst, mit 125 mg Toluolsulfonsäure-monohydrat versetzt und über Nacht bei 40°C gerührt. Die Mischung wird eingedampft, in Essigester gelöst und mit Wasser gewaschen. Der Essigester wird abdestilliert und der Rückstand aus Essigester/Cyclohexan umkristallisiert, $[\alpha]^{20}_{D}$ = +62,3° (3 % in $CHCl_3$), Smp. 119-122°C.

Beispiel 13:

Analog Beispiel 12 werden hergestellt:

a) (R)-2-Acetamido-3-methoxycarbonyl-2-penten-4-olid, $[\alpha]^{20}_{D}$ = +61,0° (3 % in $CHCl_3$), Smp. 133-134°C.

b) (S)-2-Acetamido-3-ethoxycarbonyl-2-penten-4-olid, $[\alpha]^{20}_{D}$ = -63,0° (3 % in $CHCl_3$), Smp. 121-122°C.

Beispiel 14: 2(S)-Acetamido-3(S)-methoxycarbonyl-4(R)-pentanolid

169,2 g (R)-2-Acetamido-3-methoxycarbonyl-2-penten-4-olid werden in 800 ml Methanol gelöst, mit 10 g Pd/C (5 %) versetzt und während 8 Stunden bei 30°C und 3-5 bar hydriert. Der Katalysator wird abfiltriert, mit Methanol gewaschen und das Filtrat eingedampft. Das Rohprodukt zeigt bei der HPLC-Analyse ein Diastereomerenverhältnis (2S,3S,4R):(2R,3S,4R):(2S,3R,4R) von ca. 5:3:2. Der Anteil des (2R,3R,4R)-Diastereomeren beträgt weniger als 1 %. Das Gemisch wird in 500 ml Methanol gelöst, mit 12 g 1,8-Diazabicyclo[5.4.0]undec-7-en versetzt und bei Raumtemperatur 48 Stunden gerührt, wobei ein Gemisch enthaltend 86-88 % des gewünschten (2S,3S,4R)-Diastereomeren entsteht. Die Lösung wird eingeengt, mit 2 N Salzsäure neutralisiert, in Essigester aufgenommen und mit gesättigter Kochsalzlösung extrahiert. Diastereomrenreine Titelverbindung wird durch Kristallisation aus Essigester/Cyclohexan erhalten, $[\alpha]^{20}_{D}$ = -49,5° (3 % in $CH_3OH$), Smp. 105-106°C, IR 1787, 1740, 1689, 1510 $cm^{-1}$.

Durch präparative HPLC gelingt es, aus dem Rohprodukt das (2R,3S,4R)- und das (2S,3R,4R)-Diastereomere rein zu isolieren.

Beispiel 15: 2(R)-Acetamido-3(S)-ethoxycarbonyl-4(S)-pentanolid

15 g (S)-2-Acetamido-3-ethoxycarbonyl-2-penten-4-olid werden in 100 ml Essigester gelöst, mit 0,45 g Rhodium/$Al_2O_3$-Katalysator versetzt, bei 50°C und 50 bar 21 Stunden hydriert und wie in Beispiel 14 aufgearbeitet. Das Rohprodukt zeigt bei der HPLC-Analyse ein Diastereomerenverhältnis (2R,3S,4S):(2S,3R,4S):(2R,3R,4S) von ca. 85:13:2. Diastereomerenreine Titelverbindung wird durch Kristallisation aus Essigester erhalten, $[\alpha]^{20}_{D}$ = -183,2° (3 % in $CHCl_3$), Smp. 165-167°C.

Dasselbe Diastereomere kann auch durch Verwendung von Nishimura-Katalysator (Pt/Rh) anstelle von Rh auf $Al_2O_3$ hergestellt werden.

Beispiel 16: 2(S)-Amino-3(S)-carboxy-4(R)-pentanolid

114,4 g 2(S)-Acetamido-3(S)-methoxycarbonyl-4(R)-pentanolid werden während 24 Stunden in halbkonzentrierter Salzsäure auf 120°C erhitzt. Die Lösung wird langsam auf 0°C gekühlt, der Niederschlag abfiltriert, mit Essigester gewaschen, im Hochvakuum getrocknet und aus Methanol umkristallisiert. Hydrochlorid der Titelverbindung, $[\alpha]^{20}_{D}$ = -21,5° (1 % in $H_2O$), Smp. >250°C. Durch Behandeln des Hydrochlorids mit einem Ueberschuss an Propylenoxid in heissem Methanol erhält man die freie Titelverbindung, $[\alpha]^{20}_{D}$ = -23,1° (1 % in 1 N HCl), Smp. 196-200°C.

Beispiel 17: 2(R)-Amino-3(R)-carboxy-4(S)-pentanolid

Analog Beispiel 14 und 16 erhält man die Titelverbindung aus (S)-2-Acetamido-3-ethoxycarbonyl-2-penten-4-olid. $[\alpha]^{20}_{D}$ = +22,5° (1 % in 1 N HCl), Smp. 200-204°C.

Beispiel 18: 2(S)-Amino-3(S)-methoxycarbonyl-4(R)-pentanolidhydrochlorid

19,56 g 2(S)-Amino-3(S)-carboxy-4(R)-pentanolid-hydrochlorid werden in Methanol suspendiert, während 15 Minuten mit Salzsäuregas begast und über Nacht bei Raumtemperatur gerührt. Das Lösungsmittel wird am Rotationsverdampfer abdestilliert und der Rückstand aus Chloroform kristallisiert: $[\alpha]^{20}_{D}$ = -27,3° (1 % in DMSO), Smp. 170-172°C.

Beispiel 19:

Analog Beispiel 18 werden hergestellt:

a) 2(R)-Amino-3(R)-methoxycarbonyl-4(S)-pentanolid-hydrochlorid, $[\alpha]^{20}_{D}$ = +26,7° (1 % in DMSO), Smp. 171-172°C.

b) 2(S)-Amino-3(S)-ethoxycarbonyl-4(R)-pentanolid-hydrochlorid, hergestellt in Ethanol statt in Methanol, $[\alpha]^{20}_{D}$ = -29,6° (3 % in $CHCl_3$), amorph.

Beispiel 20: 2(S)-Amino-3(R)-methoxycarbonyl-4(R)-pentanolid-hydrochlorid

4,28 g (R)-2-Amino-3-methoxycarbonyl-2-penten-4-olid (Beispiel 9) werden in 45 ml ca. 0,6 M HCl in Methanol gelöst und in Gegenwart von 1 g Rhodium auf Alox (5 %) bei 60-65°C und ca. 50 bar 7 Stunden hydriert. Der Katalysator wird abfiltriert und mit Methanol gewaschen. Das Filtrat wird eingedampft und der Rückstand aus Chloroform/Cyclohexan kristallisiert. $[\alpha]^{20}_{D}$ = +113,4° (1 % in DMSO), Smp. 178-179°C. $^1$H-NMR (DMSO): 1,26 (3H, d 6,4 Hz), 3,69 (3H, s), 3,74 (1H, dxd 7,2 und 5,3 Hz), 4,77 (1H, d 7,2 Hz), 4,92 (1H, dxq 6,4 und 5,3 Hz), 9,1 (3H, breit, $NH_3^+$).

Die Titelverbindung wird durch 1,8-Diazabicyclo[5.4.0]undec-7-en in methanol analog Beispiel 14 zum (2S,3S,4R)-Diastereomeren (Beispiel 18) epimerisiert und als Hydrochlorid aus Chloroform kristallisiert: $[\alpha]^{20}_{D}$ = -27,3° (1 % in DMSO), Smp. 170-172°C.

Beispiel 21: 2(S)-Amino-3(S)-carboxy-4(R)-hydroxy-pentansäure (β(S)-(1'(R)-Hydroxyethyl)-L-asparaginsäure)

2,07 g 2(S)-Amino-3(S)-carboxy-4(R)-pentanolid werden in 10 ml Methanol suspendiert und durch Zugabe von 13 ml 2 N wässriger Natronlauge unter Rühren bei Raumtemperatur in Lösung gebracht. Das Lösungsmittelgemisch wird abdestilliert und der ölige Rückstand mit Acetonitril versetzt. Die sich allmählich bildenden Kristalle werden abfiltriert, mit wenig Acetonitril gewaschen und am Hochvakuum getrocknet. Zurück bleibt das Dinatriumsalz der Titelverbindung, $[\alpha]_D^{20}$ = -9,0° (1 % in Wasser). $^1$H-NMR ($D_2O$): 1,21 (3H, d 6,4 Hz), 2,54 (1H, dxd 6,4 und 8,4 Hz), 3,49 (1H, d 6 Hz), 3,97 (1H, dxq 8,4 und 6,4 Hz). Die freie Säure erhält man durch Lösen des Dinatriumsalzes in einer äquivalenten Menge 1 N Salzsäure.

Beispiel 22: 2(S)-Amino-3(S)-ethoxycarbonyl-4(R)-hydroxy-pentansäure

56,0 g 2(S)-Amino-3(S)-ethoxycarbonyl-4(R)-pentanolid-hydrochlorid werden in Methylenchlorid gelöst und mit Natriumbicarbonatlösung auf pH 7,5 gestellt. Die Methylenchloridphase wird abgetrennt, die wässrige Phase mit Kochsalz gesättigt und mehrfach mit Methylenchlorid extrahiert und die organischen Phase eingedampft. Zum Rückstand in 120 ml Ethanol werden während 6 Stunden bei 0°C 106 ml 2 N Natronlauge zugetropft. Anschliessend wird die Lösung auf die Hälfte eingeengt, mit 103 ml 2 N Salzsäure auf pH 3,9 gestellt und völlig eingedampft. Der Rückstand wird aus Isopropanol/Diisopropylether kristallisiert, mit Methanol verrührt und über Kieselgel filtriert. $[\alpha]_{365}^{20}$ = +14,5° (1 % in $H_2O$), Smp. 150-151°C.

Beispiel 23: 3(S)-(1'(R)-Hydroxyethyl)-2-azetidinon-4(S)-carbonsäure

7,9 g 2(S)-Amino-3(S)-ethoxycarbonyl-4(R)-hydroxy-pentansäure werden in 40 ml Toluol suspendiert, mit 9,5 ml Hexamethyldisilazan und 2,85 ml Trimethylchlorsilan versetzt und anschliessend über Nacht am Rückfluss erhitzt. Nach dem Abkühlen wird unter Ausschluss von Feuchtigkeit unter einer Argonatmosphäre vom ausgefallenen Ammoniumchlorid abfiltriert und das Toluol abdestilliert. Zum öligen Rückstand wird ca. 1 g Ammoniakgas eingeleitet, über Nacht bei Raumtemperatur gerührt und entgast. Zurück bleibt die Bis-silylverbindung des Ausgangsmaterials, farbloses Oel, Sdp. 110°C/0,01 mbar, $[\alpha]_D^{20}$ = -11,8° (3 % in $CH_2Cl_2$).

Zu einer tert-Butylmagnesiumchlorid-Lösung in Tetrahydrofuran (45 ml, 73,2 mMol) wird bei -5°C eine Lösung von 7,75 g der obigen Bis-silylverbindung in 20 ml abs. Tetrahydrofuran innerhalb von 30 Minuten zugetropft. Anschliessend wird vorsichtig bei Raumtemperatur gerührt. Die Mischung wird 4 Stunden bei 0°C mit 10 ml Wasser hydrolysiert und mit verdünnter Phosphorsäure auf pH 4,0 angesäuert. Das Tetrahydrofuran wird abdestilliert, ausgefallenes Magnesiumhydrogenphosphat abfiltriert, die wässrige klare Lösung eingeengt, mit Phosphorsäure auf pH 2,8 und dann mit 5 M Ammoniaklösung auf pH 4,5 gestellt und nochmals filtriert. Das Filtrat wird eingedampft und der Rückstand an Kieselgel chromatographiert. Das Produkt wird aus Methanol, Isopropanol und Aceton kristallisiert, IR (DMSO): 1759, 1724 $cm^{-1}$.

Natriumsalz,

$[\alpha]_{436}^{20}$ = -3,8° (0,45 % in DMSO), IR (DMSO): 1747, 1616, 1426 $cm^{-1}$, $^1$H-NMR (DMSO): 1,14 (3H, d 6,2 Hz), 2,86 (1H, m), 3,71 (1H, d 2,5 Hz), 3,89 (1H, m), 5,08 (1H, breit, OH), 7,9 (1H, s, NH).

Beispiel 24: 4(R)-Acetoxy-3(R)-(1'(R)-hydroxyethyl)-2-azetidinon

Das Reaktionsgemisch von Beispiel 23 wird anstelle der Hydrolyse mit Wasser und Phosphorsäure zur Trockene eingedampft. 3,05 g dieses Rohprodukts mit einem Gehalt von ca. 50 % 3(S)-(1'(R)-Hydroxy ethyl)-2-azetidinon-4(S)-carbonsäure werden in 40 ml Eisessig und 5 ml Triethylamin (Wassergehalt 0,5 %) gelöst und in einer Monozelle bei einer Stromdichte von 40 mA/cm² und einer Temperatur von 30°C an Platinelektroden elektrolysiert. Der Gehalt an während der Elektrolyse gebildetem Produkt wird polarographisch verfolgt. Nach einem Stromdurchsatz von ca. 5 Faraday pro Mol wird das Gemisch in Toluol augenommen und am Rotationsverdampfer unterhalb 45° eingedampft. Das zurückbleibende Oel wird zur Abtrennung von Salzen und Nebenprodukten mit Methylenchlorid über Kieselgel filtriert und aus Toluol umkristallisiert, $[\alpha]_D^{20}$ = +63° (1 % in $CHCl_3$), Smp. 108-110°C, $R_f$ (Essigester) = 0,45. Das Rohprodukt enthält neben dem gewünschten (3R,4R,1'R)-Diastereomeren ca. 10 % des (3R,4S,1'R)-Diastereomeren (d.h. die cis-Form), $R_f$ (Essigester) = 0,35.

Beispiel 25: 4(R)-(N-Allyloxycarbonylglycylthio)-3(S)-(1'(R)-hydroxyethyl)-2-azetidinon

Eine Lösung von 426 mg N-Allyloxycarbonyl-thioglycin-dicyclohexylammoniumsalz in 1,2 ml 1 N wässriger Natronlauge wird dreimal mit 1,5 ml $CH_2Cl_2$ gewaschen und mit 0,1 N Salzsäure auf pH 8-9 gestellt. Die erhaltene wässrige Thiolsäurelösung wird bei 25°C zu einer Lösung von 173,2 mg 4(R)-Acetoxy-3(R)-(1'(R)-hydroxyethyl)-2-azetidinon in 1,7 ml Acetonitril zufliessen gelassen. Nach Zugabe von 0,1 ml 0,1 N wässriger Natronlauge wird 35 Minuten bei 21-23°C weitergerührt. Zur Aufarbeitung werden in einem Scheidetrichter 250 ml Essigsäureethylester und 30 g NaCl vorgelegt und dazu das Reaktionsgemisch gegeben. Nach gutem Schütteln und Abtrennen der wässrigen Phase wird die organische Phase noch einmal mit 50 ml 5%iger wässriger $NaHCO_3$-Lösung und zweimal mit 50 ml Sole gewaschen und über Natriumsulfat getrocknet. Das Lösungsmittel wird am Rotationsverdampfer entfernt. Man erhält die Titelverbindung als amorphes Pulver. Das Rohprodukt kann an Kieselgel chromatographisch gereinigt werden (Toluol/Essigester

2:3). $R_f$-Wert 0,23 (Merck Fertigplatten, Toluol/Essigester = 1:4, Ninhydrin als Entwicklungsreagens).

**Patentansprüche**

1. Verfahren zur Herstellung von diastereomerenreinen Verbindungen der Formel

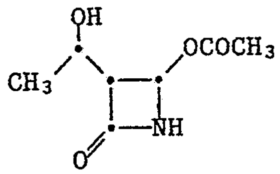

(I),

dadurch gekennzeichnet, dass man eine enantiomerenreine Verbindung der Formel

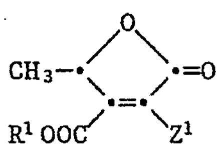

(II),

worin $R^1$ Niederalkyl und $Z^1$ Amino, Arylmethylamino, Acylamino oder Azido bedeuten, oder ein Salz davon mit Wasserstoff reduziert und gewünschtenfalls eine Acylaminogruppe $Z^1$ und/oder die Niederalkoxycarbonylgruppe $COOR^1$ hydrolysiert, in einer erhältlichen Verbindung der Formel

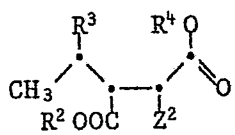

(III),

worin $R^2$ Wasserstoff oder Niederalkyl, $R^3$ und $R^4$ zusammen eine Bindung und $Z^2$ Amino oder Acylamino bedeuten, oder in einem Salz davon, gewünschtenfalls nach Umwandlung der Reste $R^2$ und $Z^2$ und/oder nach Umwandlung von Diastereomeren ineinander, mit einer Base den Lactonring öffnet und, falls $Z^2$ Acylamino bedeutet, die Acylaminogruppe $Z^2$ hydrolysiert,
in einer erhältlichen Verbindung der Formel III, worin $R^2$ Wasserstoff oder Niederalkyl, $R^3$ Hydroxy, $R^4$ Wasserstoff und $Z^2$ Amino bedeuten, oder in einem Salz davon $R^3$ und $COOR^4$ und, wenn $R^2$ Wasserstoff bedeutet, auch $COOR^2$ mit einer Schutzgruppe schützt, mit einer starken Base den β-Lactamring schliesst und die Schutzgruppen abspaltet, und
eine erhältliche Verbindung der Formel

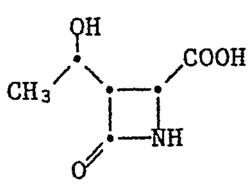

(IV)

oder ein Salz davon oxidativ in Gegenwart eines Acetat-abgebenden Mittels decarboxyliert.

2. Verfahren gemäss Anspruch 1 zur Herstellung der diastereomerenreinen Verbindung der Formel

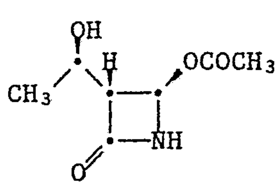

(Ia)

aus dem (R)-Enantiomeren der Verbindung der Formel II.

3. Verfahren zur Herstellung von Verbindungen der Formel

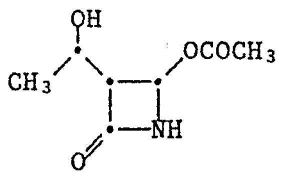

(I),

dadurch gekennzeichnet, dass eine Verbindung der Formel

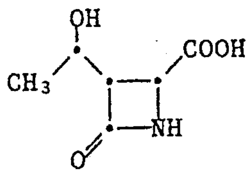

(IV)

oder ein Salz davon elektrochemisch in Gegenwart von Essigsäure und/oder Alkalimetallacetaten bei Stromdichten zwischen 10 und 400 mA/cm$^2$ und Temperaturen zwischen 0° und 50°C oxidativ decarboxyliert wird.

4. Verfahren gemäss Anspruch 3 zur Herstellung der diastereomerenreinen Verbindung der Formel

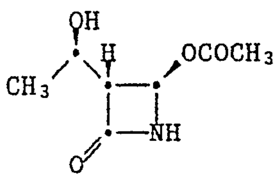

(Ia)

aus dem (3S,4S,1'R)-Diastereomeren der Verbindung der Formel IV.

5. Verfahren zur Herstellung von Verbindungen der Formel

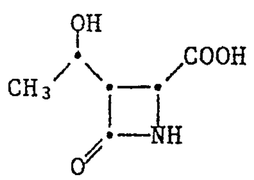

(IV),

und von Salzen dieser Verbindungen, dadurch gekennzeichnet, dass man eine Verbindung der Formel

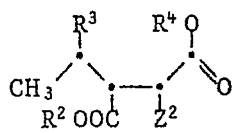

(III),

worin R$^2$ Niederalkyl, R$^3$ Hydroxy, R$^4$ Wasserstoff und Z$^2$ Amino bedeuten, oder ein Salz davon mit einem Triniederalkylsilylierungsmittel in Gegenwart einer Base in einem inerten Lösungsmittel umsetzt, die erhältliche Verbindung der Formel III, worin R$^2$ Niederalkyl, R$^3$ Triniederalkylsilyloxy, R$^4$ Triniederalkylsilyl und Z$^2$ Amino bedeuten, mit einer starken Base in einem inerten Lösungsmittel bei Temperaturen zwischen -80°C und Raumtemperatur cyclisiert und die erhältliche Verbindung hydrolysiert, und gegebenenfalls ein erhältliches Salz in die freie Verbindung oder in ein anderes Salz oder eine erhältliche freie Verbindung in ein Salz überführt.

6. Verfahren gemäss Anspruch 5, dadurch gekennzeichnet, dass als Triniederalkylsilylierungsmittel Trimethylsilylchlorid und Hexamethyldisilazan verwendet werden.

7. Verfahren gemäss Anspruch 5, dadurch gekennzeichnet, dass als starke Base ein nicht-nukleophiles Alkalimetallamid, eine in 1-Stellung verzweigte Niederalkyllithiumverbindung oder eine in 1-Stellung verzweigte Niederalkylmagnesiumverbindung verwendet wird.

8. Verfahren gemäss Anspruch 5, 6 oder 7 zur Herstellung des (3S,4S,1'R)-Diastereomeren der Verbindung der Formel IV und von Salzen dieser Verbindung aus dem entsprechenden (2S,3S,4R)-Diastereomeren der Verbindung der Formel III.

9. Verfahren zur Herstellung von Verbindungen der Formel

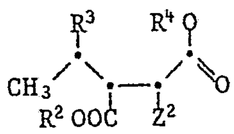

(III),

worin $R^2$ Niederalkyl, $R^3$ und $R^4$ zusammen eine Bindung und $Z^2$ Amino oder Acylamino bedeuten, und von Salzen dieser Verbindungen, dadurch gekennzeichnet, dass man eine Verbindung der Formel

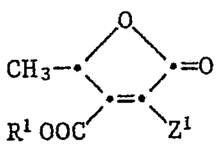

(II),

worin $R^1$ Niederalkyl und $Z^1$ Amino, Arylmethylamino, Acylamino oder Azido bedeuten, oder ein Salz davon mit Wasserstoff in Gegenwart eines Edelmetallkatalysator in einem inerten Lösungsmittel bei Temperaturen zwischen 0° und 100°C und einem Wasserstoffdruck zwischen 1 und 100 bar reduziert und gewünschtenfalls ein erhältliches Diastereomerengemisch mit einer Base in einem polaren, wasserfreien Lösungsmittel bei Temperaturen zwischen 0° und 50°C in das Diastereomere mit all-trans-Anordnung der Substituenten umwan delt, und gegebenenfalls ein erhältliches Salz in die freie Verbindung oder in ein anderes Salz oder eine erhältliche freie Verbindung in ein Salz überführt.

10. Verfahren gemäss Anspruch 9, dadurch gekennzeichnet, dass als Edelmetallkatalysator ein Platin-, Palladium- oder Platin/Rhodium-Katalysator verwendet wird.

11. Verfahren gemäss Anspruch 9 oder 10 zur Herstellung der (2S,3S,4R)-, (2R,3R,4S)-, (2R,3S,4S)-, (2S,3R,4R)-, (2R,3S,4R)- und (2S,3R,4S)-Diastereomeren der Verbindungen der Formel III und von Salzen dieser Verbindungen.

12. Verbindungen der Formel

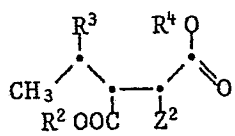

(III),

worin $R^2$ Wasserstoff, Niederalkyl oder eine Carboxyschutzgruppe, $R^3$ Hydroxy oder geschütztes Hydroxy, $R^4$ Wasserstoff oder eine Carboxyschutzgruppe oder $R^3$ und $R^4$ zusammen eine Bindung, und $Z^2$ Amino oder Acylamino bedeuten, deren reine Diastereomere und Salze davon.

13. Reine Diastereomere der Verbindungen der Formel III gemäss Anspruch 12, worin $R^2$ Wasserstoff, Niederalkyl oder eine Carboxyschutzgruppe, $R^3$ Hydroxy oder geschütztes Hydroxy, $R^4$ Wasserstoff oder eine Carboxyschutzgruppe oder $R^3$ und $R^4$ zusammen eine Bindung, und $Z^2$ Amino, Niederalkanoylamino, Niederalkoxycarbonylamino oder Arylcarbonylamino bedeuten, und Salze davon.

14. Die (2S,3S,4R)-, (2R,3R,4S)-, (2R,3S,4S)-, (2S,3R,4R)-, (2R,3S,4R)- und (2S,3R,4S)-Diastereomeren der Verbindungen der Formel III gemäss Anspruch 12, worin $R^2$ Wasserstoff, Niederalkyl oder Triniederalkylsilyl, $R^3$ Hydroxy oder Triniederalkylsilyloxy, $R^4$ Wasserstoff oder Triniederalkylsilyl oder $R^3$ und $R^4$ zusammen eine Bindung, und $Z^2$ Amino oder Niederalkanoylamino bedeuten, und Salze davon.

15. Die (2S,3S,4R)-, (2R,3R,4S)-, (2R,3S,4S)- und (2R,3S,4R)-Diastereomeren der Verbindungen der Formel III gemäss Anspruch 12, worin $R^2$ Wasserstoff oder Niederalkyl, $R^3$ und $R^4$ zusammen eine Bindung und $Z^2$ Amino oder Niederalkanoylamino bedeuten, und Salze davon.

16. Die (2S,3S,4R)-Diastereomeren der Verbindungen der Formel III gemäss Anspruch 12, worin $R^2$ Wasserstoff oder Niederalkyl, $R^3$ Hydroxy oder Triniederalkylsilyloxy, $R^4$ Wasserstoff oder Triniederalkylsilyl und $Z^2$ Amino bedeuten, und Salze davon.

17. Die Verbindung der Formel

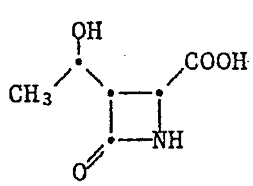

(IV)

deren reine Diastereomere und Salze dieser Verbindungen.

18. Das (3S,4S,1'R)-Diastereomere der Verbindung der Formel IV gemäss Anspruch 17 und deren Salze.

19. Reine Enantiomere der Verbindungen der Formel

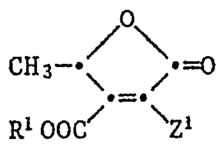

(II),

worin $R^1$ Niederalkyl und $Z^1$ Hydroxy, verethertes Hydroxy, verestertes Hydroxy, Amino, Arylmethylamino, Acylamino oder Azido bedeuten, tautomere Formen dieser Verbindungen und Salze dieser Verbindungen mit salzbildenden Gruppen.

20. Reine Enantiomere der Verbindungen der Formel II gemäss Anspruch 19, worin $R^1$ Niederalkyl und $Z^1$ Hydroxy, unsubstituiertes oder substituiertes Niederalkoxy, Aryloxy, Silyloxy, Halogen, unsubstituiertes oder substituiertes Niederalkansulfonyloxy, Arylsulfonyloxy, unsubstituiertes oder substituiertes Niederalkanoyloxy, Niederalkoxycarbonyloxy, Arylcarbonyloxy, Amino, Arylmethylamino, unsubstituiertes oder substituiertes Niederalkanoylamino, Niederalkoxycarbonylamino, Arylniederalkoxycarbonylamino, Arylcarbonylamino oder Azido bedeuten, tautomere Formen dieser Verbindungen und Salze dieser Verbindungen mit salzbildenden Gruppen.

21. Reine Enantiomere der Verbindungen der Formel II gemäss Anspruch 19, worin $R^1$ Niederalkyl und $Z^1$ Hydroxy, Halogen, unsubstituiertes oder substituiertes Niederalkansulfonyloxy, Arylsulfonyloxy, Amino, Arylmethylamino, Niederalkanoylamino, Niederalkoxycarbonylamino, Arylcarbonylamino oder Azido bedeuten, tautomere Formen dieser Verbindungen und Salze dieser Verbindungen mit salzbildenden Gruppen.

22. Reine Enantiomere der Verbindungen der Formel II gemäss Anspruch 19, worin $R^1$ Niederalkyl und $Z^1$ Hydroxy, unsubstituiertes oder substituiertes Niederalkansulfonyloxy, Arylsulfonyloxy, Amino, Arylmethylamino, Niederalkanoylamino oder Azido bedeuten, tautomere Formen dieser Verbindungen und Salze dieser Verbindungen mit salzbildenden Gruppen.

23. Verfahren zur Herstellung von enantiomerenreinen Verbindungen der Formel

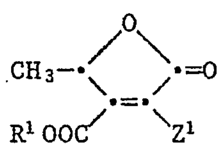

(II),

worin $R^1$ Niederalkyl und $Z^1$ Hydroxy, verethertes Hydroxy, verestertes Hydroxy, Amino, Arylmethylamino, Acylamino oder Azido bedeuten, tautomeren Formen dieser Verbindungen und Salzen dieser Verbindungen mit salzbildenden Gruppen, dadurch gekennzeichnet, dass man

a) eine enantiomerenreine Verbindung der Formel

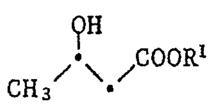

(V),

worin $R^1$ Niederalkyl bedeutet, mit einem Oxalsäurediester kondensiert, oder

b) eine racemische Verbindung der Formel II, worin $R^1$ Niederalkyl und $Z^1$ Hydroxy, Amino oder Arylmethylamino bedeuten, mit einer enantiomerenreinen, chiralen Säure oder einem reaktiven Derivat davon umsetzt, das erhältliche Diastereomerengemisch in die Diastereomeren auftrennt und, wenn erwünscht, die reinen Diastereomeren rückspaltet, oder

c) in einer prochiralen Verbindung der Formel

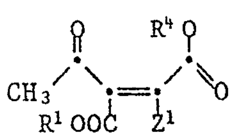

(VI),

worin $R^1$ Niederalkyl und $R^4$ Wasserstoff oder eine Carboxyschutzgruppe bedeuten und $Z^1$ die Unter Formel II genannten Bedeutungen hat, mit einem chiralen Reduktionsmittel die Carbonylgruppe reduziert und, gegebenenfalls nach Abspaltung der Schutzgruppe $R^4$, mit Säure den Lactonring schliesst, und gegebenenfalls

eine erhältliche enantiomerenreine Verbindung der Formel II in eine andere enantiomerenreine Verbindung der Formel II umwandelt und/oder ein erhältliches Salz in die freie Verbindung oder in ein anderes Salz oder eine erhältliche freie Verbindung in ein Salz überführt.

Patentansprüche für den folgenden Vertragsstaat: Es

1. Verfahren zur Herstellung von diastereomerenreinen Verbindungen der Formel

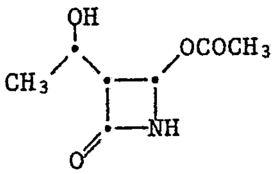

(I),

dadurch gekennzeichnet, dass man eine enantiomerenreine Verbindung der Formel

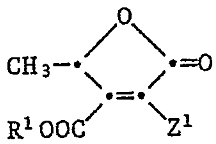

(II),

worin $R^1$ Niederalkyl und $Z^1$ Amino, Arylmethylamino, Acylamino oder Azido bedeuten, oder ein Salz davon mit Wasserstoff reduziert und gewünschtenfalls eine Acylaminogruppe $Z^1$ und/oder die Niederalkoxycarbonylgruppe $COOR^1$ hydrolysiert, in einer erhältlichen Verbindung der Formel

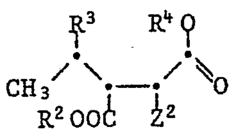

(III),

worin $R^2$ Wasserstoff oder Niederalkyl, $R^3$ und $R^4$ zusammen eine Bindung und $Z^2$ Amino oder Acylamino bedeuten, oder in einem Salz davon, gewünschtenfalls nach Umwandlung der Reste $R^2$ und $Z^2$ und/oder nach Umwandlung von Diastereomeren ineinander, mit einer Base den Lactonring öffnet und, falls $Z^2$ Acylamino bedeutet, die Acylaminogruppe $Z^2$ hydrolysiert,
in einer erhältlichen Verbindung der Formel III, worin $R^2$ Wasserstoff oder Niederalkyl, $R^3$ Hydroxy, $R^4$ Wasserstoff und $Z^2$ Amino bedeuten, oder in einem Salz davon $R^3$ und $COOR^4$ und, wenn $R^2$ Wasserstoff bedeutet, auch $COOR^2$ mit einer Schutzgruppe schützt, mit einer starken Base den β-Lactamring schliesst und die Schutzgruppen abspaltet, und
eine erhältliche Verbindung der Formel

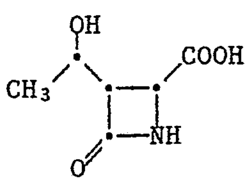

(IV)

oder ein Salz davon oxidativ in Gegenwart eines Acetat-abgebenden Mittels decarboxyliert.

2. Verfahren gemäss Anspruch 1 zur Herstellung der diastereomerenreinen Verbindung der Formel

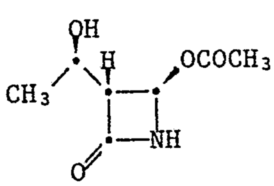

(Ia)

aus dem (R)-Enantiomeren der Verbindung der Formel II.

3. Verfahren zur Herstellung von Verbindungen der Formel

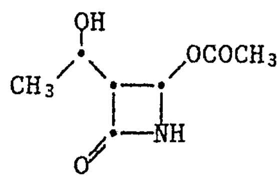

(I),

dadurch gekennzeichnet, dass eine Verbindung der Formel

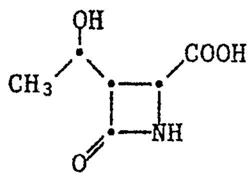

(IV)

oder ein Salz davon elektrochemisch in Gegenwart von Essigsäure und/oder Alkalimetallacetaten bei Stromdichten zwischen 10 und 400 mA/cm$^2$ und Temperaturen zwischen 0° und 50°C oxidativ decarboxyliert wird.

4. Verfahren gemäss Anspruch 3 zur Herstellung der diastereomerenreinen Verbindung der Formel

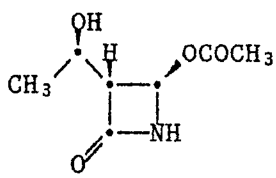

(Ia)

aus dem (3S,4S,1'R)-Diastereomeren der Verbindung der Formel IV.

5. Verfahren zur Herstellung von Verbindungen der Formel

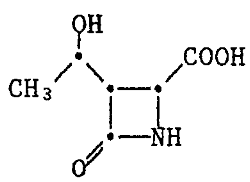

(IV),

und von Salzen dieser Verbindungen, dadurch gekennzeichnet, dass man eine Verbindung der Formel

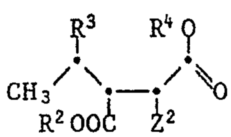

(III),

worin R$^2$ Niederalkyl, R$^3$ Hydroxy, R$^4$ Wasserstoff und Z$^2$ Amino bedeuten, oder ein Salz davon mit einem Triniederalkylsilylierungsmittel in Gegenwart einer Base in einem inerten Lösungsmittel umsetzt, die erhältliche Verbindung der Formel III, worin R$^2$ Niederalkyl, R$^3$ Triniederalkylsilyloxy, R$^4$ Triniederalkylsilyl und Z$^2$ Amino bedeuten, mit einer starken Base in einem inerten Lösungsmittel bei Temperaturen zwischen -80°C und Raumtemperatur cyclisiert und die erhältliche Verbindung hydrolysiert, und gegebenenfalls ein erhältliches Salz in die freie Verbindung oder in ein anderes Salz oder eine erhältliche freie Verbindung in ein Salz überführt.

6. Verfahren gemäss Anspruch 5, dadurch gekennzeichnet, dass als Triniederalkylsilylierungsmittel Trimethylsilylchlorid und Hexamethyldisilazan verwendet werden.

7. Verfahren gemäss Anspruch 5, dadurch gekennzeichnet, dass als starke Base ein nicht-nukleophiles Alkalimetallamid, eine in 1-Stellung verzweigte Niederalkyllithiumverbindung oder eine in 1-Stellung verzweigte Niederalkylmagnesiumverbindung verwendet wird.

8. Verfahren gemäss Anspruch 5, 6 oder 7 zur Herstellung des (3S,4S,1'R)-Diastereomeren der Verbindung der Formel IV und von Salzen dieser Verbindung aus dem entsprechenden (2S,3S,4R)-Diastereomeren der Verbindung der Formel III.

9. Verfahren zur Herstellung von Verbindungen der Formel

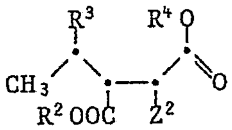 (III),

worin $R^2$ Niederalkyl, $R^3$ und $R^4$ zusammen eine Bindung und $Z^2$ Amino oder Acylamino bedeuten, und von Salzen dieser Verbindungen, dadurch gekennzeichnet, dass man eine Verbindung der Formel

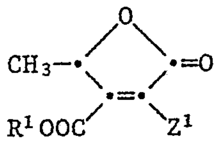 (II),

worin $R^1$ Niederalkyl und $Z^1$ Amino, Arylmethylamino, Acylamino oder Azido bedeuten, oder ein Salz davon mit Wasserstoff in Gegenwart eines Edelmetallkatalysator in einem inerten Lösungsmittel bei Temperaturen zwischen 0° und 100°C und einem Wasserstoffdruck zwischen 1 und 100 bar reduziert und gewünschtenfalls ein erhältliches Diastereomerengemisch mit einer Base in einem polaren, wasserfreien Lösungsmittel bei Temperaturen zwischen 0° und 50°C in das Diastereomere mit all-trans-Anordnung der Substituenten umwandelt, und gegebenenfalls ein erhältliches Salz in die freie Verbindung oder in ein anderes Salz oder eine erhältliche freie Verbindung in ein Salz überführt.

10. Verfahren gemäss Anspruch 9, dadurch gekennzeichnet, dass als Edelmetallkatalysator ein Platin-, Palladium- oder Platin/Rhodium-Katalysator verwendet wird.

11. Verfahren gemäss Anspruch 9 oder 10 zur Herstellung der (2S,3S,4R)-, (2R,3R,4S)-, (2R,3S,4S)-, (2S,3R,4R)-, (2R,3S,4R)- und (2S,3R,4S)-Diastereomeren der Verbindungen der Formel III und von Salzen dieser Verbindungen.

12. Verfahren gemäss Anspruch 9 oder 10 zur Herstellung der (2S,3S,4R)-, (2R,3R,4S)-, (2R,3S,4S)- und (2R,3S,4R)-Diastereomeren der Verbindungen der Formel III, worin $R^2$ Niederalkyl, $R^3$ und $R^4$ zusammen eine Bindung und $Z^2$ Amino oder Niederalkanoylamino bedeuten, and von Salzen dieser Verbindungen.

13. Verfahren zur Herstellung von enantiomerenreinen Verbindungen der Formel

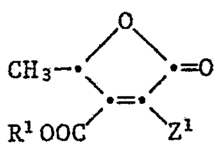 (II),

worin $R^1$ Niederalkyl und $Z^1$ Hydroxy, verethertes Hydroxy, verestertes Hydroxy, Amino, Arylmethylamino, Acylamino oder Azido bedeuten, tautomeren Formen dieser Verbindungen und Salzen dieser Verbindungen mit salzbildenden Gruppen, dadurch gekennzeichnet, dass man

a) eine enantiomerenreine Verbindung der Formel

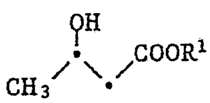 (V),

worin $R^1$ Niederalkyl bedeutet, mit einem Oxalsäurediester kondensiert, oder

b) eine racemische Verbindung der Formel II, worin $R^1$ Niederalkyl und $Z^1$ Hydroxy, Amino oder Arylmethylamino bedeuten, mit einer enantiomerenreinen, chiralen Säure oder einem reaktiven Derivat davon umsetzt, das erhältliche Diastereomerengemisch in die Diastereomeren auftrennt und, wenn erwünscht, die reinen Diastereomeren rückspaltet, oder

c) in einer prochiralen Verbindung der Formel

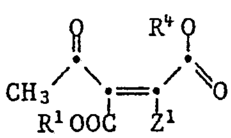 (VI),

worin $R^1$ Niederalkyl und $R^4$ Wasserstoff oder eine Carboxyschutzgruppe bedeuten und $Z^1$ die unter

Formel II genannten Bedeutungen hat, mit einem chiralen Reduktionsmittel die Carbonylgruppe reduziert und, gegebenenfalls nach Abspaltung der Schutzgruppe $R^4$, mit Säure den Lactonring schliesst, und gegebenenfalls
eine erhältliche enantiomerenreine Verbindung der Formel II in eine andere enantiomerenreine Verbindung der Formel II umwandelt und/oder ein erhältliches Salz in die freie Verbindung oder in ein anderes Salz oder eine erhältliche freie Verbindung in ein Salz überführt.

14. Verfahren gemäss Anspruch 13 zur Herstellung von enantiomerenreinen Verbindungen der Formel II, worin $R^1$ Niederalkyl und $Z^1$ Hydroxy, unsubstituiertes oder substituiertes Niederalkoxy, Aryloxy, Silyloxy, Halogen, unsubstituiertes oder substituiertes Niederalkansulfonyloxy, Arylsulfonyloxy, unsubstituiertes oder substituiertes Niederalkanoyloxy, Niederalkoxycarbonyloxy, Arylcarbonyloxy, Amino, Arylmethylamino, unsubstituiertes oder substituiertes Niederalkanoylamino, Niederalkoxycarbonylamino, Arylniederalkoxycarbonylamino, Arylcarbonylamino oder Azido bedeuten, tautomeren Formen dieser Verbindungen und Salzen dieser Verbindungen mit salzbildenden Gruppen.

15. Verfahren gemäss Anspruch 13 zur Herstellung von enantiomerenreinen Verbindungen der Formel II, worin $R^1$ Niederalkyl und $Z^1$ Hydroxy, Halogen, unsubstituiertes oder substituiertes Niederalkansulfonyloxy, Arylsulfonyloxy, Amino, Arylmethylamino, Niederalkanoylamino, Niederalkoxycarbonylamino, Arylcarbonylamino oder Azido bedeuten, tautomeren Formen dieser Verbindungen und Salzen dieser Verbindungen mit salzbildenden Gruppen.

16. Verfahren gemäss Anspruch 13 zur Herstellung von enantiomerenreinen Verbindungen der Formel II, worin $R^1$ Niederalkyl und $Z^1$ Hydroxy, unsubstituiertes oder substituiertes Niederalkansulfonyloxy, Arylsulfonyloxy, Amino, Arylmethylamino, Niederalkanoylamino oder Azido bedeuten, tautomeren Formen dieser Verbindungen und Salzen dieser Verbindungen mit salzbildenden Gruppen.